# EUROPEAN PATENT APPLICATION

(11) **EP 3 424 511 A1**
(43) Date of publication of application: **09.01.2019**
(21) Application number: 18154976.7
(22) Date of filing: 14.05.2013
(51) Int. Cl.: A61K 31/713

(54) **MULTI-TARGET MODULATION FOR TREATING FIBROSIS AND INFLAMMATORY CONDITIONS**

(30) Priority: 16.05.2012 US 201261648076 P
(62) Divisional of application: 13791023.8
(71) Applicant: Aadigen, LLC, Pacific Palisades, CA 90272 (US)
(72) Inventor: DESAI, Neil, Pacific Palisades, CA 90272 (US)
(74) Representative: J A Kemp

(57) **Abstract**

The present invention relates to compositions comprising one or more active agents that selectively modulate the expression of two or more genes, for example at the post-transcription level, that are involved in fibrosis and/or inflammatory conditions. Also provided are methods of using such compositions for treating fibrotic diseases, as well as other diseases including inflammatory diseases and cancer.

## Description

### CROSS-REFERENCE TO RELATED APPLICATION

This patent application claims priority to U.S. Provisional Patent Application No. 61/648,076, filed May 16, 2012, the disclosure of which is incorporated herein by reference in its entirety.

### TECHNICAL FIELD

This application relates generally to the fields of compositions and methods for treating fibrosis and inflammatory compositions by modulating the expression of two or more target genes.

### BACKGROUND

Fibrosis is the formation o-f excess fibrous connective tissue in an organ or tissue in a reparative or reactive process, in a pathological situation, as opposed to formation of fibrous tissue as a normal constituent of an organ or tissue. Fibrosis may be the result of chronic inflammatory reactions induced by a variety of stimuli including persistent infections, autoimmune reactions, allergic responses, chemical insults, radiation, and tissue injury. Scarring is confluent fibrosis that obliterates the architecture of the underlying organ or tissue. Different organs can be affected by fibrotic diseases, including: Pulmonary fibrosis (lungs); Idiopathic pulmonary fibrosis (where the cause is unknown), Cystic fibrosis (caused by genetic mutation of CFTR gene [cystic fibrosis transmembrane conductance regulator]); Cirrhosis (liver); Endomyocardial fibrosis (heart); Progressive kidney disease, Mediastinal fibrosis (soft tissue of the mediastinum); Myelofibrosis (bone marrow), Retroperitoneal fibrosis (soft tissue of the retroperitoneum); Progressive massive fibrosis (lungs); a complication of coal workers' pneumoconiosis; Nephrogenic systemic fibrosis (skin), Crohn's Disease (intestine); Keloid (skin); Old myocardial infarction (heart); Scleroderma/systemic sclerosis (skin, lungs); Arthrofibrosis (knee, shoulder, other joints); and some forms of adhesive capsulitis (shoulder).

Despite having distinct clinical manifestations, most chronic fibrotic disorders have in common a persistent irritant that sustains the production of growth factors, proteolytic enzymes, angiogenic factors and fibrogenic cytokines, which stimulate the deposition of connective tissue elements that progressively remodel and destroy normal tissue architecture [1-3]. In some diseases, such as idiopathic pulmonary fibrosis, liver cirrhosis, cardiovascular fibrosis, systemic sclerosis, scleroderma and nephritis, extensive tissue remodelling and fibrosis can ultimately lead to organ failure and death.

Idiopathic pulmonary fibrosis, scleroderma and liver fibrosis/cirrhosis are serious fibrosis-related diseases that present significant clinical problems and unmet medical needs for which to date there are no approved or effective drugs.

Idiopathic pulmonary fibrosis (IPF): IPF is a progressive and generally fatal disease characterized by scarring of the lungs that thickens the lung lining, causing an irreversible loss of lung structure and function. IPF affects 5 million people worldwide and 130,000-200,000 people in the US, with about 48,000 new cases diagnosed and 40,000 deaths each year, with medical costs estimated at $2.8 billion per 100,000 patients [2, 3]. Median survival is 2-4 years following diagnosis, and the 5-year survival is 20-40% [4, 5]. Currently, there is no known cause, no FDA approved treatments and no cure for IPF [6]. IPF patients typically are treated with anti-inflammatory drugs, including corticosteroids and cytotoxic agents, despite the fact that there is no evidence that they have any effect on long-term patient survival.

While drugs currently under investigation for IPF including IFNgamma, Etanercept, Bosentan, and Pirfenidone, QAX576 (IL-13 blocker), FG-3019 (CTGF antibody), CNTO-888 (CCL2 antibody), GC-1008 (TGF beta-antibody), Losartan and Thalidomide are directed against targets in the pathology of IPF, concerns remain about their clinical efficacy as a single agent therapy due to the redundancy of signaling pathways involved in IPF [6],

Systemic Scleroderma (SS): SS is an autoimmune disorder and a connective tissue disease that involves changes in the skin, blood vessels, muscles, and internal organs. There are an estimated 300,000 people in the US who have scleroderma, a third of whom have SS [7]. Local disease affects only skin tissues while SS affects skin and underlying tissues, blood vessels, and major organs including the heart, lungs, or kidneys. In diffuse cutaneous disease, five-year survival is 70%, and 10-year survival is 55% [8]. Currently there is no cure and no specific treatment for scleroderma. SS treatment includes immunosuppressive and anti-inflammatory drugs including corticosteroids, methotrexate, cyclophosphamide, azathioprine, and mycophenolate [9, 10].

Liver cirrhosis (LC): Liver fibrogenesis is characterized by excessive accumulation of extracellular matrix (ECM), leading to cirrhosis and complications including portal hypertension, liver failure, and hepatocellular carcinoma [11]. LC causes 800,000 deaths worldwide annually [12]. In the US, LC causes 27,000 deaths annually [13]. Established cirrhosis has a 10-year mortality of 34-66% [14]. Common causes of cirrhosis include alcohol consumption, chronic hepatitis B, C and D, obesity, toxins, bile duct diseases, and autoimmune hepatitis [15]. Currently there is no treatment available for LC, and health care costs for managing this disease are high.

Tissue fibrosis is defined by the overgrowth, hardening, and/or scarring of various tissues as a result of excessive deposition of extracellular matrix (ECM). The key cellular mediator of fibrosis is the myofibroblast, which produces excessive amounts of ECM components such as collagen when activated (Claman, 1991). Myofibroblasts are generated from a variety of sources including resident mesenchymal cells, epithelial and endothelial cells in processes termed epithelial/endothelial-mesenchymal (EMT/EndMT) transition, as well as from circulating fibroblast-like cells called fibrocytes that are derived from bone-marrow stem cells. Myofibroblasts are activated by a variety of mechanisms, including paracrine signals derived from lymphocytes and macrophages, autocrine factors secreted by myofibroblasts, and pathogen-associated molecular patterns produced by pathogenic organisms recognized by pattern recognition receptors (i.e. TLRs) on fibroblasts.

The ECM contains 3 major components: structural proteins and proteoglycans, non-structural matricellular proteins, and growth factors/inflammatory cytokines (Brekken, 2001). Although structural proteins such as collagens are most prominently increased in fibrotic tissues, matricellular proteins and growth factors are believed to be the major players in the maintenance of homeostasis in the ECM. Numerous studies have shown that ECM biosynthesis and deposition are regulated by matricellular proteins and growth factors and by alterations in cell-ECM interactions that are accompanied by reorganization of the cytoskeletal network (Varedi, 1997).

The progression of fibrotic diseases involves the intricate interplay of all 3 ECM components. Growth factors, cytokines and chemokines stimulate the proliferation and activation of lymphocytes, macrophages and myofibroblasts. Activated myofibroblasts and other cells express high levels of different ECM proteins, including matricellular proteins and other non-structural proteins, and structural proteins such as collagen, fibronectin, and vitronectin. Cytokines (IL-13, IL-21, TGF-beta1), chemokines (MCP-1, MIP-1beta), angiogenic factors (VEGF), growth factors (PDGF, CTGF), peroxisome proliferator-activated receptors (PPARs), acute phase proteins (SAP), caspases, and components of the renin-angiotensin-aldosterone system (ANG II) have been identified as important regulators of fibrosis (Wynn, 2008).

The matricellular and other non-structural proteins play key roles in the organization and remodeling of ECM. Several such proteins have been closely associated with the pathogenesis of fibrosis including SPARC, thrombospondin, CCN1, LOXL2, TGF-beta1, CTGF, and TIMP.

The expression and deposition of ECM proteins in fibrotic diseases are regulated by complicated cascades of cell surface receptors, signaling molecules, and transcription factors such as. Smad 3, Smad 7, SOX9, arrestin.

RNAi is an evolutionarily conserved process of sequence-specific, post-transcriptional gene silencing. RNAi is initiated by dsRNA that is homologous in sequence to the silenced gene. MicroRNAs (miRNAs) are a class of endogenous, small, non-coding RNA molecules of ∼22 nucleotides that are located in independent noncoding transcripts or in introns of protein-coding genes and post-transcriptionally control the translation and stability of mRNAs. Small interfering RNAs (siRNA) are double-stranded RNA molecules approximately 21-25 base pairs (bp) long that act to inhibit gene expression through initiating enzymatic degradation of a sequence-matched mRNA. miRNAs and siRNAs bind to the RNA-induced silencing complex (RISC), which selectively retains the antisense strand (guide strand) and silences gene expression by degrading the complementary and corresponding mRNA strand.

While the targeted gene down regulation by RNAi is proposed as a potential therapeutic strategy for a multitude of disease conditions, the safe and effective delivery of RNAi therapeutics remains a challenge. The physicochemical characteristics of siRNA and miRNA (i.e. large molecular weight and anionic charge) prevent passive diffusion across the plasma membrane of most cell types, and siRNA is prone to degradation in physiological conditions. Therefore a biocompatible, nontoxic and nonimmunogenic carrier is required to deliver siRNA and miRNA to the target site, which will dramatically improve its clinical potential. The current siRNA and miRNA delivery methods include viral transfection or non-viral techniques including liposomes, dendrimers, linear polymers, polymerosomes, micelles, peptides, nanoparticles, and local delivery by electrotransfer.

All references cited herein, including patent applications and publications, are incorporated by reference in their entirety.

### SUMMARY OF THE INVENTION

The present invention provides compositions comprising one or more (such as two or more) active agents that selectively modulate the expression of two or more genes, for example at the post-transcription level, that are involved in fibrosis and/or inflammatory conditions. Also provided are methods of using such compositions for treating fibrotic diseases, as well as other diseases including inflammatory diseases and cancer.

The present invention in some embodiments provides a pharmaceutical composition for preventing or treating fibrosis and other diseases involving chronic inflammation in mammals by targeting 2 or more genes involved in the development and progression of fibrosis using one or more nucleic acids (such as oligonucleotides) including, but not limited to, antisense oligonucleotides, RNAi (such as shRNAs, siRNAs, miRNAs, antagomirs) and plasmid DNA.

The present invention also provides a pharmaceutical composition to treat fibrosis or inflammatory conditions or diseases, including but not limited to: pulmonary fibrosis, idiopathic pulmonary fibrosis, progressive massive fibrosis of the lung, cystic fibrosis, mediastinal fibrosis, liver fibrosis, cirrhosis, endomyocardial fibrosis, cardiac fibrosis, old myocardial infarction, progressive kidney disease, renal fibrosis, myelofibrosis, retroperitoneal fibrosis, nephrogenic systemic fibrosis, Crohn's disease, keloids, scleroderma/systemic sclerosis, arthrofibrosis, adhesive capsulitis, fibromyalgia, peritoneal fibrosis, radiation induced fibrosis, burn induced fibrosis, trauma induced fibrosis, scarring fibrosis, and wound healing fibrosis..

The invention compositions are also useful for wound healing resulting from traumatic injury such as burns or for healing of chronic wounds and reducing scarring and fibrosis

The present invention also provides a pharmaceutical composition to treat desmoplastic cancers or cancers having a fibrotic component, including but not limited to: squamous cell carcinomas independent of their location, bilio-pancreatic carcinomas, mesothelioma, desmoplastic fibroma, desmoplastic round cell tumor, breast cancer, ovarian cancer, colorectal carcinoma and tumors of gastrointestinal tract, lung cancers, lymphomas, myelofibrosis, leukemias melanoma, brain tumors (including glioblastoma, cerebral astrocytoma, neuroblastoma, and medulloblastoma), bladder cancers, hepatocellular and urothelial tumors, and tumors of the pituitary gland.,

The present invention provides a pharmaceutical composition that comprises multiple (two or more) single stranded or double stranded nucleic acids, including antisense oligonucleotides, RNAi, shRNAs, siRNAs, miRNAs, antagomirs or plasmid DNA which inhibits or modulate the activity or expression of two or more of genes involved in the development and progression of fibrosis, including but not limited to: CTGF, TGFbeta1, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP- 1 GAD65, sGAD65, BAX, p53 PTEN, STAT5, smoothened, GLI1, GLI2, and Patched-1. Yet another gene of interest for the present invention is HIF-1alpha.

The present invention provides a pharmaceutical composition in which multiple (two or more) single stranded or double stranded nucleic acids including antisense oligonucleotides, shRNAs, siRNAs, miRNAs, antagomirs, plasmid DNA ,etc. are covalently conjugated to, or non-covalently associated with, or formulated with peptides, proteins, antibodies, lipids, phospholipids, polymers, aptamers, nanoparticles, liposomes, dendrimers, polymerosomes, viral vectors, and micelles. Such a composition is suitable for mammalian and human use.

The present invention provides a method of treatment and a method to administer to human and other mammals an effective amount of a pharmaceutical composition once or multiple times to treat fibrosis and other inflammatory conditions or diseases by systemic or local administration. The administration routes include but are not limited to: oral, intravenous, intraarterial, intracardiac, intracatheter, intraperitoneal, intravesical, transdermal, nasal inhalation, pulmonary delivery, intracavity, intracranial, intrathecal, subcutaneous, intradermal, intramuscular, intraocular, topical, rectal, vaginal, direct injection/administration, and local delivery with electrotransfer or microneedle injection.

In one preferred embodiment, the single stranded or double stranded nucleic acids including antisense oligonucleotides, shRNAs, siRNAs, miRNAs, antagomirs, plasmid DNA etc. are encapsulated in a peptide-based nanoparticle and administered via intravenous systemic administration.

Invention compositions maybe prepared in powder form, liquid form, tablet or capsule form, in the form of gels, creams, ointments, sprays, embedded in wound dressings or dissolving strips and are suitable for administration by the methods described above

Devices used for delivery can include delivery through a needle, microneedle, convection enhanced delivery device, catheters, intravesical urinary catheters, aerosol, inhaler, nasal spray, pessary, suppository, single use or repeat use devices, creams, ointments, patches, etc.

The present invention comprises a method to select effective combinations of 2 or more gene targets or corresponding expressed proteins for down regulation or modulation. These gene targets can be selected from different aspects implicated in the development of fibrosis, including but not limited to ECM components (structural proteins and proteoglycans, non-structural matricellular proteins, and growth factors/inflammatory cytokines), and cellular components of fibroblasts (receptors for growth factors/inflammatory cytokines, signal transduction molecules such as kinases, enzymes, and adaptor proteins, transcription factors, and other cellular components regulating the proliferation, metabolism, survival, migration, and differentiation of fibroblasts).

### DETAILED DESCRIPTION

In recent years, multiple proteins have been implicated as potential key regulators promoting pathogenesis of fibrotic diseases, and some studies have suggested that targeted down regulation of an individual gene through the RNAi approach could have therapeutic effect against fibrosis. However, little effort has been made to develop a multi-target RNAi (shRNA, siRNA and/or miRNA,) or plasmid DNA therapeutics to prevent and treat fibrotic diseases by inhibiting the expression of two or more genes involved in fibrosis. The present invention provides a therapeutic (for example a composition comprising multiple nucleic acids such as oligonucleotides, particularly siRNA or miRNA molecules) aiming for the combined inhibition of two or more target genes involved in fibrosis.

The present invention thus in some embodiments relates to the use of a combination of multiple single or double stranded nucleic acids (such as antisense oligonucleotides, RNAi, shRNAs, siRNAs, miRNAs, antagomirs and plasmid DNA) for selectively modulating (such as inhibiting) the expression of two or more genes, for example at the post-transcription level, whereas these genes controls the same or distinct signaling pathways and cellular procedures, and play essential roles in the pathology and progression of fibrosis diseases affecting different organs. The invention also provides the methods of composition and delivering the combination of nucleic acids (such as oligonucleotides) to the fibrotic disease sites in target organs, including the use of a novel peptide-based nanoparticle oligonucleotide delivery vehicle as a carrier of the combination of oligonucleotide compounds for the treatment of fibrotic diseases. The invention also describes the therapeutic use of such compositions containing oligonucleotide compounds to treat patients with fibrotic diseases, as well as other diseases including inflammatory diseases and cancer.

In some embodiments, the composition comprises two or more active agents in a predetermined ratio. This allows simultaneous delivery of multiple active agents, which may modulate the expression of different targets. The predetermined ratio can be selected so as to allow the most effective delivery and/or target modulation.

The present application therefore in one aspect provides a pharmaceutical composition comprising one or more nucleic acids (such as oligonucleotides) that specifically target the expression of two or more genes involved in the development or progression of fibrosis or inflammation. In another aspect, there is provided a pharmaceutical composition comprising one or more active agents that modulate the activity or expression of two or more genes involved in the development and progression of fibrosis or inflammation, selected from, CTGF(CCN2), TGFbeta1, TGFbeta receptor 1, TGFbeta receptor 2, TGFbeta receptor 3, beta -catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5 and relevant genes in the hedgehog pathway - smoothened, GLI1, GLI2, and Patched-1. Yet another gene of interest for the present invention is HIF-1alpha,

In another aspect, there is provided a method of treating a fibrotic or inflammatory condition in an individual comprising administering to said individual an effective amount of any of the pharmaceutical composition described herein. Also provided are kits, unit dosages, and articles of manufacture useful for the methods described herein.

It is understood that aspect and embodiments of the invention described herein include "consisting" and/or "consisting essentially of" aspects and embodiments.

As used herein, the singular form "a", "an", and "the" includes plural references unless indicated otherwise.

As is understood by one skilled in the art, reference to "about" a value or parameter herein includes (and describes) embodiments that are directed to that value or parameter per se. For example, description referring to "about X" includes description of "X".

### I. Methods and compositions of the present invention

In some embodiments, there is provided a method of treating a fibrotic or inflammatory condition in an individual, comprising administering to the individual an effective amount of a pharmaceutical composition comprising one or more (such as any of 2, 3, 4, 5, 6, 7, or 8) nucleic acids (for example single-stranded or double-stranded oligonucleotides) that modulate the expression of two or more genes involved in the development or progression of fibrosis or inflammation. In some embodiments, the two or more genes are selected from the group consisting of CTGF(CCN2), TGFbeta1, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, and relevant genes in the hedgehog pathway - smoothened, GLI1, GLI2, and Patched-1, Yet another gene of interest for the present invention is HIF-1alpha. In some embodiments, at least one of the nucleic acids is selected from the group consisting of antisense oligonucleotide, RNAi, shRNA, siRNA, miRNA, or plasmid DNA.

In some embodiments, there is provided a method of treating a fibrotic or inflammatory condition in an individual, comprising administering to the individual an effective amount of a pharmaceutical composition comprising one or more (such as any of 2, 3, 4, 5, 6, 7, or 8) active agents (such as nucleic acids, for example, single-stranded or double-stranded oligonucleotides) that modulate the activity or expression of two or more genes involved in the development and progression of fibrosis or inflammation, selected from, CTGF(CCN2), TGFbeta1, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, and relevant genes in the hedgehog pathway - smoothened, GLI1, GLI2, and Patched-1. Yet another gene of interest for the present invention is HIF-lalpha. In some embodiments, at least one of the active agents is a protein (such as an antibody). In some embodiments, at least one of the active agents is a small molecule. In some embodiments, at least one of the active agents is an oligonucleotide. In some embodiments, the two active agents are of the same kind. In some embodiments, the two active agents are of different kinds.

In some embodiments, there is provided a method of treating a fibrotic or inflammatory condition in an individual, comprising administering to the individual an effective amount of a pharmaceutical composition comprising one or more (such as any of 2, 3, 4, 5, 6, 7, or 8) RNAi (for example siRNA or shRNA) that modulate the expression of two or more genes involved in the development or progression of fibrosis or inflammation. In some embodiments, the two or more genes are selected from the group consisting of CTGF(CCN2), TGFbetat, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7,, SOX9, arrestin, PDCD4, PAI-1, NF-kB, and PARP-1 GAD65, sGAD65, BAX, p53 PTEN, STAT5, and relevant genes in the hedgehog pathway - smoothened, GLI1, GLI2, and Patched-1. Yet another gene of interest for the present invention is HIF-1alpha. In some embodiments, the nucleic acids are about 10 to about 50 nucleotides long. In some embodiments, the two or more nucleic acids are of the same kind. In some embodiments, the two or more nucleic acids are of different kinds.

The fibrotic or inflammatory condition described herein in some embodiments is selected from the group consisting of pulmonary fibrosis, idiopathic pulmonary fibrosis, progressive massive fibrosis of the lung, cystic fibrosis, mediastinal fibrosis, liver fibrosis, cirrhosis, endomyocardial fibrosis, cardiac fibrosis, old myocardial infarction, progressive kidney disease, renal fibrosis, myelofibrosis, retroperitoneal fibrosis, nephrogenic systemic fibrosis, Crohn's disease, keloids, scleroderma/systemic sclerosis, arthrofibrosis, adhesive capsulitis, fibromyalgia, peritoneal fibrosis, radiation induced fibrosis, burn induced fibrosis, trauma induced fibrosis, scarring fibrosis, and wound healing fibrosis. In some embodiments, the fibrotic or inflammatory conditions is a desmoplastic cancer, wherein the desmoplastic cancer is selected from the group consisting of: squamous cell carcinoma independent of their location, bilio-pancreatic carcinoma, mesothelioma, desmoplastic fibroma, desmoplastic round cell tumor, breast cancer, ovarian cancer, colorectal carcinoma and tumor of gastrointestinal tract, lung cancer, lymphoma, myelofibrosis, leukemia, melanoma, brain tumor (including glioblastoma, cerebral astrocytoma, neuroblastoma, and medulloblastoma), bladder cancer, hepatocellular and urothelial tumor, and tumor of the pituitary gland. The pharmaceutical composition can be administered by any of suitable route, including, but not limited to, oral, intravenous, intraarterial, intracardiac, intracatheter, intraperitoneal, intravesical, transdermal, nasal inhalation, pulmonary delivery, intracavity, intracranial, intrathecal, subcutaneous, intradermal, intramuscular, intraocular, topical, rectal, vaginal, direct injection/administration, or local delivery with electrotransfer or microneedle injection.

In some embodiments according to any one of the methods described above, the individual for treatment is selected based on the expression level of at least one genes involved in the development and progression of fibrosis or inflammation. In some embodiments, the method further comprises determining the expression level of at least one gene prior to the administration of the pharmaceutical composition.

"Modulation" of activity or expression means regulating or altering the status or copy numbers of a gene or mRNA or changing the amount of gene product such as a protein that is produced. In some embodiments, the active agent inhibits the expression of the target gene. In some embodiments, the modulation (such as inhibition) occurs at a post-transcriptional level. In some embodiments, the active agent inhibits the expression of the gene or gene product by at least about any of 10%, 20%, 30%, 40%, 60%, 70%, 80%, 90%, or 100%. In some embodiments such as in the case of Plasmid delivery, the active agent may increase the expression of the gene or gene product by at least about any of 10%, 20%, 30%, 40%, 60%, 70%, 80%, 90%, or 100%.

In another aspect, there are provided pharmaceutical compositions comprising two or more (such as any of 2, 3, 4, 5, 6, 7, or 8) active agents (such as nucleic acids, for example single-stranded or double-stranded oligonucleotides) that modulate the expression of two or more genes involved in the development or progression of fibrosis or inflammation, including, but not limited to, CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, and relevant genes in the hedgehog pathway - smoothened, GLI1, GLI2, and Patched-1. Yet another gene of interest for the present invention is HIF-1alpha. These pharmaceutical compositions described herein are useful for any one of the methods described herein. In some embodiments, the two or more active agents are of the same kind. In some embodiments, the two or more active agents are of different kinds. In some embodiments, the pharmaceutical composition comprises two active agents, and wherein the molar ratio of the two active agents is about 0.1:1 to about 10:1. In some embodiments, the two or more active agents in the pharmaceutical composition are in equal molar proportions.

In some embodiments, there are provided pharmaceutical compositions comprising two or more (such as any of 2, 3, 4, 5, 6, 7, or 8) RNAi (such as siRNA or shRNA) that modulate the expression of two or more genes involved in the development or progression of fibrosis or inflammation, including, but not limited to, CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, and relevant genes in the hedgehog pathway - smoothened, GLI1, GLI2, and Patched-1. In some embodiments, the two interfering RNAs are of the same kind. In some embodiments, the two or more interfering RNAs are of different kinds. In some embodiments, the pharmaceutical composition comprises two interfering RNAs, and wherein the molar ratio of the two active agents is about 0.1:1 to about 10:1. In some embodiments, the two or more interfering RNAs in the pharmaceutical composition are in equal molar proportions.

The nucleic acids described in any of the embodiments above can be of any length between about 12 to about 100 nucleotides long, including for example about any of 15-80, 18-60, 20-50, or 25-30 nucleotides long. In some embodiments, the nucleic acids are at least about 60% (including for example at least about any of 70%, 80%, 90%, 95%, 98%, or 99%) identical to the corresponding target gene. In some embodiments, the nucleic acids are modified, for example by incorporating non-naturally occurring nucleotides.

The active agents (such as nucleic acids) described in any of the embodiments above can be associated (such as covalently or non-covalently associated) with a carrier molecule, which can include, but are not limited to, a peptide, a protein, an antibody, a lipid, a phospholipid, a polymer, an aptamer, a nanoparticle, a liposome, a dendrimer, a polymerosome, a viral vector, a micelle or synthetic compositions including of any of the above that can interact with the with the active agents through charge interactions and/or hydrophobic interactions, e.g. a molecule containing charged species and a lipid, or a molecule containing a charged peptide and a lipid. In some embodiments, the carrier molecule is a peptide (such as a cell-penetrating peptide, for example a polycationic peptide or an amphipathic peptide). Suitable cell-penetrating peptides include, but are not limited to, a PTD-based peptide, an amphipathic peptide, a poly-arginine-based peptide, a MPG peptide, a CADY peptide, or a Pep-1 or Pep-2, peptide. In some embodiments, the composition comprises two or more different types of peptides (such as different types of cell-penetrating peptides). For example, in some embodiments, the composition comprises both polycationic peptide and amphipathic peptide. In some embodiments, the composition comprises a MPG peptide and a CADY peptide. Other combinations of the carrier peptides are also contemplated.

The nucleic acids and the cell-penetrating peptide in some embodiments can be present in a complex. In some embodiments, the nucleic acids and the cell-penetrating peptides are present in nanoparticles comprising the complexes of the nucleic acids and the cell-penetrating peptide. The average size of the nanoparticles can be between any of about 30 nm and about 10 microns, including for example between about 50 nm and about 1 micron, between about 50 nm and about 400 nm. The molar ratio of the cell-penetrating peptide and the nucleic acids in the composition can be about 100:1 to about 1:50, including about any of 50:1 to 1:20, 20:1 to 1:10, and the like.

The compositions and methods described in this section are further described below in more detail.

### II. Pharmaceutical compositions comprising two or more active agents

Active agents described herein include nucleic acid based molecules such as oligonucleotides, nucleic acids, polynucleotides, single or double stranded oligo and polynucleotides, antisense oligonucleotides, different forms of RNAi such as siRNA, shRNA, etc., microRNA (miRNA), antagomirs, ribozyme, aptamer, plasmid DNA, etc. and suitable combinations of one or more of these agents. In addition, active agents may also include proteins such as enzymes or antibodies having a specific desired effect or small molecules with desired specific activity. Also contemplated are combinations of nucleic acid based agents with proteins or small molecules that are either covalently attached to each other or provided without any covalent attachment as suitable combinations.

Target genes: Specific and preferred active agents and their targets include those that are directed towards fibrosis and inflammation. These include agents directed to CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, and STAT5, and relevant genes in the hedgehog pathway - smoothened, GLI1, GLI2, and Patched-1. Yet another gene of interest for the present invention is HIF-1alpha. The specific agents of interest acting on these targets include nucleic acid based molecules, proteins, enzymes or antibodies, and small molecules and their combinations as described above. Most preferred are compositions which contain more than one active agent thereby having the ability to simultaneously affect or modulate more than one target related to fibrosis or inflammation.

Preferred molecular targets and key signaling pathways implicated in the pathology of fibrotic diseases, such as Idiopathic Pulmonary Fibrosis, Systemic Scleroderma, and Liver Cirrhosis include transforming growth factor (TGF)-beta and its receptors, connective tissue growth factor (CTGF), adenosine receptors, SPARC (secreted protein acidic and rich in cysteine) and tissue inhibitors of metalloproteinases (TIMPs), which are involved in the activation of fibroblasts, inflammatory responses, ECM deposition, and tissue repair.

The active agent(s) described herein may modulate the expression of two or more genes in the same signaling pathway, or they modulate the expression of two or more genes in different pathways. For example, in some embodiments, the active agent (such as nucleic acids, for example RNAi) modulates the expression of the TGF-beta signaling pathway. In some embodiments, the active agent (such as nucleic acids, for example RNAi) modulate the expression of the CTGF signaling pathway. In some embodiments, the active agent (such as nucleic acid, for example RNAi) modulates the expression of a protein involved in the hedgehog pathway (which includes, but is not limited to, Patched-1, smoothened, GLI1, GLI-2). In some embodiments, the active agent (such as nucleic acid, for example RNAi) modulates the expression of an ECM protein (which includes, but is not limited to, collagen, fibronectin, and vitronectin). In some embodiments, the active agent (such as nucleic acid, for example RNAi) modulates the expression of a non-structural protein involved in the organization and remodeling of the ECM (which includes, but is not limited to, SPARC, thrombospondin, CCN1, LOXL2, TGF-beta-1, CTGF, and TIMP). In some embodiments, the active agent (such as nucleic acid, for example RNAi) modulates the expression of a regulator of fibrosis (which includes, but is not limited to, cytokines, chemokines, angiogenic factors, growth factors, PPAPs, SAP, caspases, and components of the renin-angiotensin-aldosterone system (ANG II).

In some embodiments, the active agent(s) modulate the expressions of two or more targets proteins in the same signaling transduction pathway. In some embodiments, the active agent(s) modulate the expression of target proteins in two or more different signal transduction pathways. The active agent(s) can modulate any combinations of the targets described above. For example, In some embodiments, the active agent (such as nucleic acid, for example RNAi) modulates the expression of both the TGF-beta signaling pathway and the CTGF pathway or both the SPARC and the CTGF pathway . Other target combinations include, but are not limited to, the transforming growth factor (TGF)-beta receptors 1 or 2 with CTGF, transforming growth factor (TGF)-beta receptors 1 or 2 with SPARC, transforming growth factor (TGF)-beta receptors 1 or 2 with Adenosine receptors, transforming growth factor (TGF)-beta receptors 1 or 2 with TIMPs, SPARC with CTGF, SPARC with Adenosine receptors, SPARC with TIMPs, CTGF with Adenosine receptors, CTGF with TIMPs, Adenosine receptors with TIMPs, Triple combinations of targets include any of the double combinations indicated above with additional single targets mentioned above.

In some embodiments, at least one of target genes is a gene involved in the TGF-beta signaling pathway, including TGF-beta . The action of TGF-beta is characterized by increased production of ECM components, as well as mesenchymal cell proliferation, differentiation, migration, and accumulation [16, 17]. TGF-beta also induces production of CTGF, which promotes fibrogenesis and causes nodular fibrosis in the liver [18, 19]. In lung fibroblasts, TGF-beta up regulates the expression of SPARC, which is a key modulator of ECM organization and overexpressed in IPF [20].

In some embodiments, at least one of target genes is a gene involved in the CTGF signaling pathway, including CTGF . CTGF is involved in radiation-induced fibrosis, lung fibrosis, and scleroderma [18]. Transgenic mice overexpressing CTGF in fibroblasts display multiorgan fibrosis similar to fibrosis observed with scleroderma [21, 22]. CTGF siRNA reduce mRNA and protein levels of SPARC, CTGF, Collagen 1 and TGF-beta1 in fibroblasts in vitro, and attenuates bleomycin induced fibrosis in skin and lungs [23].

In some embodiments, at least one of target genes is a gene involved in the adenosine signaling pathway, including adenosine receptors A2A and A2B. Adenosine and its receptors A2A and A2B in pathological conditions promote fibrosis in the skin, lungs, and liver [24]. A2B receptor overexpression is observed in surgical lung biopsies from severe chronic obstructive pulmonary disease (COPD) and IPF patients [25]. Adenosine, by acting at A2A receptors, stimulates hepatic stellate cell-mediated fibrosis of the liver by increasing production of collagen I and III [26, 27].

In some embodiments, at least one of target genes is a gene involved in the SPARC signaling pathway, including SPARC . SPARC, a matricellular protein, promotes fibrogenesis in IPF, scleroderma, and liver fibrosis [28-30]. SPARC is a key downstream mediator of TGF-beta1 activity [31], and its expression is up regulated by TGF-beta via the PI3k signaling pathway in IPF fibroblasts [20]. SPARC in turn also regulates the expression of TGF-beta1[32]. Skin and lung fibrosis induced by bleomycin in mice was markedly reduced by SPARC siRNA delivered by subcutaneous injection and intratracheal instillation, respectively [29]. Liver fibrosis in rats treated with thiocetamide was significantly attenuated by treatment with SPARC antisense [33].

In some embodiments, at least one of target genes is a gene involved in the TIMP (tissue inhibitors of metalloproteinase) signaling pathway, including TIMP . Tissue inhibitors of metalloproteinases (TIMPs) are the endogenous inhibitors of the matrix metalloproteinase families. TIMP-1 inhibits the majority of the MMPs while TIMP-3 inhibits all known interstitial and membrane-bound MMPs [34]. TIMPs play a pivotal role in liver fibrogenesis [35], and lung tissue samples from IPF patients display high expression of TIMPs, supporting the hypothesis that TIMPs contribute to a nondegrading fibrillar collagen microenvironment [36].

The methods and compositions described herein allow for modulation of the expression of two or more target genes. Exemplary target combinations include, but are not limited to, the transforming growth factor (TGF)-beta receptors 1 or 2 with CTGF, transforming growth factor (TGF)-beta receptors 1 or 2 with SPARC, transforming growth factor (TGF)-beta receptors 1 or 2 with Adenosine receptors, transforming growth factor (TGF)-beta receptors 1 or 2 with TIMPs, SPARC with CTGF, SPARC with Adenosine receptors, SPARC with TIMPs, CTGF with Adenosine receptors, CTGF with TIMPs, Adenosine receptors with TIMPs, Triple combinations of targets include any of the double combinations indicated above with additional single targets mentioned above.

Nucleic acids: In some embodiments, the active agents present in the pharmaceutical compositions are nucleic acids (such as oligonucleotides).

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. The term "nucleic acid" as used herein refers to a polymer containing at least two deoxyribonucleotides or ribonucleotides in either single- or double-stranded form and includes DNA and RNA. DNA may be in the form of, e.g., antisense molecules, plasmid DNA, pre-condensed DNA, a PCR product, vectors (P1, PAC, BAC, YAC, artificial chromosomes), expression cassettes, chimeric sequences, chromosomal DNA, or derivatives and combinations of these groups. RNA may be in the form of siRNA, asymmetrical interfering RNA (aiRNA), microRNA (miRNA), mRNA, tRNA, rRNA, tRNA, viral RNA (vRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides. "Oligonucleotide," as used herein, generally refers to short, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

In some embodiments, the nucleic acids are single stranded oligonucleotides. In some embodiments, the nucleic acids are double stranded oligonucleotides. The nucleic acids described herein may be any of a range of length of up to, but not necessarily 200 nucleotides in the case of antisense oligonucleotides, RNAi, siRNA, shRNA, miRNA, antagomirs or up to 1000 kilo bases in the case of Plasmid DNA.

In some embodiments, the nucleic acids are interference RNA, such as siRNA or shRNA. "The term "interfering RNA" or "RNAi" or "interfering RNA sequence" refers to single-stranded RNA (e.g., mature miRNA) or double-stranded RNA (i.e., duplex RNA such as siRNA, aiRNA, or pre-miRNA) that is capable of reducing or inhibiting the expression of a target gene or sequence (e.g., by mediating the degradation or inhibiting the translation of mRNAs which are complementary to the interfering RNA sequence) when the interfering RNA is in the same cell as the target gene or sequence. Interfering RNA thus refers to the single-stranded RNA that is complementary to a target mRNA sequence or to the double-stranded RNA formed by two complementary strands or by a single, self-complementary strand. Interfering RNA may have substantial or complete identity to the target gene or sequence, or may comprise a region of mismatch (i.e., a mismatch motif). The sequence of the interfering RNA can correspond to the full-length target gene, or a subsequence thereof. Interfering RNA includes "small-interfering RNA" or "siRNA," e.g., interfering RNA of about 15-60, 15-50, or 15-40 (duplex) nucleotides in length, more typically about 15-30, 15-25, or 19-25 (duplex) nucleotides in length, and is preferably about 20-24, 21-22, or 21-23 (duplex) nucleotides in length (e.g., each complementary sequence of the double-stranded siRNA is 15-60, 15-50, 15-40, 15-30, 15-25, or 19-25 nucleotides in length, preferably about 20-24, 21-22, or 21-23 nucleotides in length, and the double-stranded siRNA is about 15-60, 15-50, 15-40, 15-30, 15-25, or 19-25 base pairs in length, preferably about 18-22, 19-20, or 19-21 base pairs in length). siRNA duplexes may comprise 3' overhangs of about 1 to about 4 nucleotides or about 2 to about 3 nucleotides and 5' phosphate termini. Examples of siRNA include, without limitation, a double-stranded polynucleotide molecule assembled from two separate stranded molecules, wherein one strand is the sense strand and the other is the complementary antisense strand; a double-stranded polynucleotide molecule assembled from a single stranded molecule, where the sense and antisense regions are linked by a nucleic acid-based or non-nucleic acid-based linker; a double-stranded polynucleotide molecule with a hairpin secondary structure having self-complementary sense and antisense regions: and a circular single-stranded polynucleotide molecule with two or more loop structures and a stem having self-complementary sense and antisense regions, where the circular polynucleotide can be processed in vivo or in vitro to generate an active double-stranded siRNA molecule. Preferably, siRNA are chemically synthesized. siRNA can also be generated by cleavage of longer dsRNA (e.g., dsRNA greater than about 25 nucleotides in length) with the E. coli RNase III or Dicer. These enzymes process the dsRNA into biologically active siRNA (see, e.g., Yang et al., Proc. Natl. Acad. Sci. USA, 99:9942-9947 (2002); Calegari et al., Proc. Natl. Acad. Sci. USA, 99:14236 (2002); Byrom et al., Ambion TechNotes, 10(1):4-6 (2003); Kawasaki et al., Nucleic Acids Res., 31:981-987 (2003); Knight et al., Science, 293:2269-2271 (2001); and Robertson et al., J. Biol. Chem., 243:82 (1968)). Preferably, dsRNA are at least 50 nucleotides to about 100, 200, 300, 400, or 500 nucleotides in length. A dsRNA may be as long as 1000, 1500, 2000, 5000 nucleotides in length, or longer. The dsRNA can encode for an entire gene transcript or a partial gene transcript In certain instances, siRNA may be encoded by a plasmid (e.g., transcribed as sequences that automatically fold into duplexes with hairpin loops). A small hairpin RNA or short hairpin RNA (shRNA) is a sequence of RNA that makes a tight hairpin turn that can be used to silence gene expression via RNA interference. The shRNA hairpin structure is cleaved by the cellular machinery into siRNA, which is then bound to the RNA-induced silencing complex (RISC). This complex binds to and cleaves mRNAs which match the siRNA that is bound to it. Suitable length of the interference RNA are about 5 to about 200 nucleotides, or 10-50 nucleotides or base pairs or 15-30 nucleotides or base pairs. In some embodiments, the interference RNA is substantially complementary (such as at least about 60%, 70%, 80%, 90%, 95%, 98%, 99%, or more identical to) the corresponding target gene. In some embodiments, the interference RNA is modified, for example by incorporating non-naturally occurring nucleotides. In some embodiments the targets and corresponding sequences are indicated below:

| Target | siRNA sense sequence |
|---|---|
| Adenosine receptor A2A | 5'-GCUGAAGCAGAUGGAGAGCCA-3' |
| Adenosine receptor A2B | 5'-GCUACACUUUUCACAAAAUUA-3' |
| Angiotensin receptor 1 | 5'-ATTGTCCCAAAGCTGGAAGGC-3' |
| Angiotensin receptor 2 | 5'-UUGAUAGGUACCAAUCUGUCA-3' |
| Arrestin beta 1 | 5'-AUUACCAUGGAGAACCCAUCA-3' |
| Beta-catenin | 5'-CGGGAUGUUCACAACCGAAUU-3' |
| CCL2 | 5'-AUUAAUACAAAGAAUUUUUUU-3' |
| CCN1 | 5'-GUUACCAAUGACAACCCUGAG-3' |
| CTGF | 5'-CCGGAGACAAUGACAUCUUUG-3' |
| | OR |
| | 5'-GCACCAGUGUGAAGACAUA-3' |
| HSP47 | 5'-ACGAGAAGGAAAAGCUGCAAA-3' |
| LOXL2 | 5'-ACCAAAGUGUACAAAAUGUUU-3' |
| MMP2 | 5'-UUGAUGCGGUAUACGAGGCCC-3' |
| MMP9 | 5'-GGCGACCUCAAGUGGCACCAC-3' |
| | PAI-1 5'-AGUUCAACUAUACUGAGUUCA-3' |
| PARP-1 | 5'-AUAACCGAAGAUUGCUGUGGC-3' |
| PDCD4 | 5'-AUAGAGAGUGACAUCUAAGC-3' |
| PTEN | 5'-UGUAAUGAUAUGUGCAUAUUU-3' |
| Smad 3 | 5'-UGUUCCAGUGUGUCUUAGAGA-3' |
| Smad 4 | 5'-GUUACUGUUGAUGGAUACGUG-3' |
| Smad 7 | 5'-CACUUCAAACUACUUUGCUGC-3' |
| SOX9 | 5'-AGAGAGGACCAACCAGAAUUC-3' |
| SPARC | 5'-AACAAGACCUUCGACUCUUCCC-3' |
| | OR |
| | 5'-GCACCACACGUUUCUUUG-3' |
| STAT5A | 5'-GUUGUGAGUUUAGUAAGGCUG-3' |
| TGF beta receptor 1 | 5'-CUAUGCAAlJGGGCUUAGUAUU-3' |
| TGF beta receptor 2 | 5'-AAUGACGAGAACAUAACACUA-3' |
| TGF beta receptor 3 | 5'-UAACAAUUGAUAUAAGACCUU-3' |
| TGF beta 1 | 5'-UUAAAAGUGGAGCAGCACGUG-3' |
| Thrombospondin 1 | 5'-GCACGUGGUGUCUGUGGAGA-3' |
| TIMP-1 | 5'-AAGGGUUCCAAGCCUUAGGGG-3' |
| TIMP-3 | 5'-CUGCAAGAUCAAGUCCUGCUA-3' |
| VEGF | 5'-AUGUGAAUGCAGAUGUGACAA-3' |

In some embodiments, the nucleic acids are double-stranded antisense RNA. Suitable length of the interference RNA are about 5 to about 200 nucleotides, or 10-50 nucleotides or base pairs or 15-30 nucleotides or base pairs. In some embodiments, the interference RNA is substantially complementary (such as at least about 60%, 70%, 80%, 90%, 95%, 98%, 99%, or more identical to) the corresponding target gene. In some embodiments, the antisense RNA is modified, for example by incorporating non-naturally occurring nucleotides.

In some embodiments, the nucleic acids are miRNA. A microRNA (abbreviated miRNA) is a short ribonucleic acid (RNA) molecule found in eukaryotic cells. A microRNA molecule has very few nucleotides (an average of 22) compared with other RNAs. miRNAs are post-transcriptional regulators that bind to complementary sequences on target messenger RNA transcripts (rnRNAs), usually resulting in translational repression or target degradation and gene silencing. The human genome may encode over 1000 miRNAs, which may target about 60% of mammalian genes and are abundant in many human cell types. Suitable length of the miRNAs are about 5 to about 200 nucleotides, or 10-50 nucleotides or base pairs or 15-30 nucleotides or base pairs. In some embodiments, the miRNA is substantially complementary (such as at least about 60%, 70%, 80%, 90%, 95%, 98%, 99%, or more identical to) the corresponding target gene. In some embodiments, the antisense RNA is modified, for example by incorporating non-naturally occurring nucleotides.

In some embodiments, the nucleic acids are plasmid DNA or DNA fragments (for example DNA fragments of lengths of up to about 1000 bp). In addition, the plasmid DNA or DNA fragments may be hypermethylated or hypomethylated.

Carrier molecules: The active agents described herein in some embodiments are associated with carrier molecules. Carrier molecules in some embodiments can be selected from the group consisting of a peptide, a protein, an antibody, a lipid, a phospholipid, a polymer, polycationic polymers, an aptamer, a nanoparticle, a liposome, a dendrimer, a polymerosome, a viral vector, or a micelle. The carrier molecules can be combined with the desired active agents of interest to be delivered. The combinations may include covalent or non-covalent interactions between the carriers and the active agents.

In some embodiments, the carrier molecule is a peptide, such as a cell penetrating peptide. "Cell-penetrating peptide" as used herein refers to short peptides that facilitate cellular uptake of various molecular cargo (from nanosize particles to small chemical molecules and large fragments of DNA). The "cargo" is associated with the peptides either through chemical linkage via covalent bonds or through non-covalent interactions. The function of the CPPs are to deliver the cargo into cells, a process that commonly occurs through endocytosis with the cargo delivered to the endosomes of living mammalian cells. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. In some embodiments, the cell-penetrating peptide and the active agents are combined to form complexes or nanoparticles.

Suitable cell-penetrating peptides include those disclosed in US 7906484 and its counterparts, the contents of which are included herein by reference in their entirety. Peptides of interest are those identified by the formula GLFXALLXLLXSLWXLLLXAZ₁Z₂Z₃Z₄ wherein each X is independently E, R, A, I, L, F, P, W, V, N, C, Q, G, S, T or Y, and wherein each of Z₁, Z₂, Z₃, and Z₄, is independently H, K, R, or Q. Specific sequences include GLFRALLRLLRSLWRLLLRAHHHH and GLFRALLRLLRSLWRLLLRARRRR. Other peptides of interest are those identified in US 6841535, US 7514530, US 7579318, US 7943581, , in Deshayes et al (2012), Small, DOI: 10.1002/smll.201102413, and in Rydstrom et al. (2011), PLoS ONE 6(10): e25924. doi:10.1371/journal.pone.0025924, the contents of which are included herein by reference in their entirety. Peptides of interest include for example the peptides Ac-GLWRALWRLLRSLWRLLWKA-cysteamide and corresponding modifications including Ac-XLWRALWRLXRSLWRLLWKA-cysteamide [where X= G, L, beta-A, S, L, W, C or I], or Ac-XWRSXGWRWRXLWRWXXWXR-cysteamide [where X= L , S, G, beta-A, W, C or I]. In addition any of the peptides above can be stapled to improve their activity as is described by Zhang et al (2011) [[Zhang H, Curreli F, Zhang X, Bhattacharya S, Waheed AA, Cooper A, Cowburn D, Freed EO, Debnath AK. (2011) Antiviral activity of alpha-helical stapled peptides designed from the HIV-1 capsid dimerization domain. Retrovirology. 2011 May 3; 8:28. ]], the contents of which are incorporated herein by reference in their entirety.

Additional cell-penetrating peptides suitable for invention use are disclosed in WO 2012/137150 A2 and WO 2012/137036 A1 both of which are incorporated by reference herein in their entirety. These include peptides designated as the VEPEP-6 series and stapled peptides designated as the ST-VEPEP-6 series and modifications thereof.

In the case of lipid based carriers, these are described in US 8058069 or US8034376, the contents of which are incorporated herein by reference in their entirety. The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids. A "lipid particle" is used herein to refer to a lipid formulation that can be used to deliver an active agent or therapeutic agent, such as a nucleic acid (e.g., an interfering RNA), to a target site of interest. In the lipid particle of the invention, which is typically formed from a cationic lipid, a non-cationic lipid, and a conjugated lipid that prevents aggregation of the particle, the active agent or therapeutic agent may be encapsulated in the lipid, thereby protecting the agent from enzymatic degradation. As used herein, the term "SNALP" refers to a stable nucleic acid-lipid particle. A SNALP represents a particle made from lipids (e.g., a cationic lipid, a non-cationic lipid, and a conjugated lipid that prevents aggregation of the particle), wherein the nucleic acid (e.g., siRNA, aiRNA, miRNA, ssDNA, dsDNA, ssRNA, short hairpin RNA (shRNA), dsRNA, or a plasmid, including plasmids from which an interfering RNA is transcribed) is fully encapsulated within the lipid. As used herein, the term "SNALP" includes an SPLP, which is the term used to refer to a nucleic acid-lipid particle comprising a nucleic acid (e.g., a plasmid) encapsulated within the lipid. SNALP and SPLP typically contain a cationic lipid, a non-cationic lipid, and a lipid conjugate (e.g., a PEG-lipid conjugate). SNALP and SPLP are extremely useful for systemic applications, as they can exhibit extended circulation lifetimes following intravenous (i.v.) injection, they can accumulate at distal sites (e.g., sites physically separated from the administration site), and they can mediate expression of the transfected gene or silencing of target gene expression at these distal sites. SPLP include "pSPLP," which comprise an encapsulated condensing agent-nucleic acid complex as set forth in PCT Publication No. WO 00/03683, the disclosure of which is herein incorporated by reference in its entirety for all purposes.

Other suitable carriers include ones such as ECHO (Wang et al, 2009, MOLECULAR PHARMACEUTICS VOL. 6, NO. 3, 38-746) and their modifications. EHCO (1-aminoethyl) iminobis[N-(oleicylcysteinylhistinyl-1-aminoethyl)propionamide], shows pH sensitive amphiphilic cell membrane disruption. EHCO forms stable nanoparticles with siRNA. Targeted siRNA delivery systems are readily formed by surface modification of the nanoparticles. PEGylation of the siRNA/EHCO nanoparticles significantly reduces nonspecific cell uptake. The incorporation of a bombesin peptide or RGD peptide via a PEG spacer results in receptor mediated cellular uptake and high gene silencing efficiency in U87 cells. Fluorescence confocal microscopic studies demonstrate that EHCO/siRNA nanoparticles and PEG modified EHCO/ siRNA nanoparticles are able to facilitate endosomal escape of the siRNA delivery systems. Systemic administration of a therapeutic anti-HIF-1R siRNA with the peptide-targeted delivery systems results in significant tumor growth inhibition than a nontargeted delivery system or free siRNA via intravenous injection in nude mice bearing human glioma U87 xenografts. Modifications of ECHO are also useful for this purpose.

Pharmaceutical compositions: Preferred compositions of the present invention have more than one nucleic acid based active agent. In case where 2 active agents are used, they are present in molar ratios of about 1:9 to 9:1, more preferably in the range of about 3:7 to about 7:3, and 4:6 to about 6:4 and most preferably at a ratio of 1:1. In the case where 3 active agents are used, the preferred ranges of each active agent are in the molar proportion of 1:1:8 to 8:1:1, or in the range of 2:2:6 to 6:2:2, or in the range of 3:3:4 to 4:3:3 or present in equal proportions. In the case where more 4 active agents are used, it is preferred to use them in the range of molar proportion of 1:1:1:7 to 7:1:1:1, or in the range of 2:2:2:4 to 4:2:2:2 or have them present in equal proportions. When more than 4 active agents are used, it is preferred to have them present in equal molar proportions.

The peptide-based particles of the invention typically have a mean diameter of from about 1 0 nm to about 300 nm, from about 50 nm to about 200 nm, from about 60 nm to about 150 nm, from about 70 nm to about 110 nm, or from about 70 to about 90 nm, and are substantially non-toxic.

Preferably, the molar ratio (peptide):(nucleic acid) in the complex is between 1:100 and 100:1, more preferably between 1:1 and 80:1, yet more preferably between 5:1 and 30:1, most preferably between 8:1 and 20:1, and specifically 10:1, 15:1 or 20:1. The optimal molar ratio is easily determined by the skilled person, for example as described herein below. Specifically, the skilled person can test the transfection efficiency of complexes having a variety of molar ratios (peptide):(nucleic acid) for any given peptide/nucleic acid combination, and will chose the ratio giving the best transfection efficiency.

The lipid particles of the invention (e.g., SNALP) typically have a mean diameter of from about 40 nm to about 150 nm, from about 50 nm to about 150 nm, from about 60 nm to about 130 nm, from about 70 nm to about 110 nm, or from about 70 to about 90 nm, and are substantially non-toxic. In addition, nucleic acids, when present in the lipid particles of the invention, are resistant in aqueous solution to degradation with a nuclease. Nucleic acid-lipid particles and their method of preparation are disclosed in, e.g., U.S. Patent Publication Nos. 20040142025 and 20070042031, US8034376, the disclosures of which are herein incorporated by reference in their entirety for all purposes.

Targeting: According to a particular embodiment, the complex of the invention further comprise at least one ligand capable of cell-specific and/or nuclear targeting. A cell membrane surface receptor is a molecule or structure which can bind said ligand with high affinity and preferably with high specificity. Said cell membrane surface receptor is preferably specific for a particular cell, i.e. it is found predominantly in one type of cell rather than in another type of cell (e.g. galactosyl residues to target the asialoglycoprotein receptor on the surface of hepatocytes). The cell membrane surface receptor facilitates cell targeting and internalization into the target cell of the ligand (i.e. the peptide involved in cell-specific targeting) and attached molecules (i.e. the complex of the invention). A large number of ligand moieties/ ligand binding partners that may be used in the context of the present invention are widely described in the literature. Such a ligand moiety is capable of conferring to the complex of the invention, the ability to bind to a given binding-partner molecule or a class of binding-partner molecules localized at the surface of at least one target cell. Suitable binding-partner molecules include without limitation polypeptides selected from the group consisting of cell-specific markers, tissue-specific markers, cellular receptors, viral antigens, antigenic epitopes and tumor-associated markers. Binding-partner molecules may moreover consist of or comprise one or more sugar, lipid, glycolipid or antibody molecules. According to the invention, a ligand moiety may be for example a lipid, a glycolipid, a hormone, a sugar, a polymer (e.g. PEG, polylysine, PET), an oligonucleotide, a vitamin, an antigen, all or part of a lectin, all or part of a polypeptide such as for example JTS1 (WO 94/40958), an antibody or a fragment thereof, or a combination thereof. Preferably, the ligand moiety used in the present invention is a peptide or polypeptide having a minimal length of 7 amino acids. It is either a native polypeptide or a polypeptide derived from a native polypeptide. "Derived" means containing (a) one or more modifications with respect to the native sequence (e.g. addition, deletion and/or substitution of one or more residues), (b) amino acid analogs, including not naturally occurring amino acids or (c) substituted linkages or (d) other modifications known in the art. The polypeptides serving as ligand moiety encompass variant and chimeric polypeptides obtained by fusing sequences of various origins, such as for example a humanized antibody which combines the variable region of a mouse antibody and the constant region of a human immunoglobulin. In addition, such polypeptides may have a linear or cyclized structure (e.g. by flanking at both extremities a polypeptide ligand by cysteine residues). Additionally, the polypeptide in use as ligand moiety may include modifications of its original structure by way of substitution or addition of chemical moieties (e.g. glycosylation, alkylation, acetylation, amidation, phosphorylation, addition of sulfhydryl groups and the like). The invention further contemplates modifications that render the ligand moiety detectable. For this purpose, modifications with a detectable moiety can be envisaged (i.e. a scintigraphic, radioactive, or fluorescent moiety, or a dye label and the like). Suitable radioactive labels include but are not limited to .sup.99Tc, .sup.123I, and .sup.111In. Such detectable labels may be attached to the ligand moiety by any conventional techniques and may be used for diagnostic purposes (e.g. imaging of tumoral cells). In one special embodiment, the binding-partner molecule is an antigen (e.g. a target cell-specific antigen, a disease-specific antigen, an antigen specifically expressed on the surface of engineered target cells) and the ligand moiety is an antibody, a fragment or a minimal recognition unit thereof (i.e. a fragment still presenting an antigenic specificity) such as those described in detail in immunology manuals (see for example Immunology, third edition 1993, Roitt, Brostoff and Male, ed Gambli, Mosby). The ligand moiety may be a monoclonal antibody. Monoclonal antibodies which will bind to many of these antigens are already known but in any case, with today's techniques in relation to monoclonal antibody technology, antibodies may be prepared to most antigens. The ligand moiety may be a part of an antibody (for example a Fab fragment) or a synthetic antibody fragment (for example, ScFv). According to an advantageous embodiment, the ligand moiety is selected among antibody fragments, rather than whole antibodies. Effective functions of whole antibodies, such as complement binding, are removed. ScFv and dAb antibody fragments may be expressed as a fusion with one or more other polypeptides. Minimal recognition units may be derived from the sequence of one or more of the complementary-determining regions (CDR) of the Fv fragment. Whole antibodies, and F(ab')2 fragments are "bivalent". By "bivalent" we mean that said antibodies and F(ab') 2 fragments have two antigen binding sites. In contrast, Fab, Fv, ScFv, dAb fragments and minimal recognition units are monovalent, having only one antigen binding sites. In a preferred embodiment, the ligand moiety allows to target a tumor cell and is capable of recognizing and binding to a molecule related to the tumor status, such as a tumor-specific antigen, a cellular protein differentially or over-expressed in tumor cells or a gene product of a cancer-associated virus. Examples of tumor-specific antigens include but are not limited to MUC-1 related to breast cancer (Hareuveni et al., 1990, Eur. J. Biochem 189, 475-486), the products encoded by the mutated BRCA1 and BRCA2 genes related to breast and ovarian cancers (Miki et al., 1994, Science 226, 66-71; Futreal et al., 1994, Science 226, 120-122; Wooster et al., 1995, Nature 378, 789-792), APC related to colon cancer (Polakis, 1995, Curr. Opin. Genet. Dev. 5, 66-71), prostate specific antigen (PSA) related to prostate cancer, (Stamey et al., 1987, New England J. Med. 317, 909), carcinoma embryonic antigen (CEA) related to colon cancers (Schrewe et al., 1990, Mol. Cell. Biol. 10, 2738-2748), tyrosinase related to melanomas (Vile et al., 1993, Cancer Res. 53, 3860-3864), receptor for melanocyte-stimulating hormone (MSH) which is expressed in high number in melanoma cells, ErbB-2 related to breast and pancreas cancers (Harris et al., 1994, Gene Therapy 1, 170-175), and alpha-foetoprotein related to liver cancers (Kanai et al., 1997, Cancer Res. 57, 461-465). A special ligand moiety in use in the present invention is a fragment of an antibody capable of recognizing and binding to the MUC-1 antigen and thus targeting the MUC-1 positive tumor cells. A more preferred ligand moiety is the scFv fragment of the SM3 monoclonal antibody which recognizes the tandem repeat region of the MUC-1 antigen (Burshell et al., 1987, Cancer Res. 47, 5476-5482; Girling et al., 1989, Int J. Cancer 43, 1072-1076; Dokurno et al., 1998, J. Mol. Biol. 284, 713-728). Examples of cellular proteins differentially or overexpressed in tumor cells include but are not limited to the receptor for interleukin 2 (IL-2) overexpressed in some lymphoid tumors, GRP (Gastrin Release Peptide) overexpressed in lung carcinoma cells, pancreas, prostate and stomach tumors (Michael et al., 1995, Gene Therapy 2, 660-668), TNF (Tumor Necrosis Factor) receptor, epidermal growth factor receptors, Fas receptor, CD40 receptor, CD30 receptor, CD27 receptor, OX-40, alpha.-v integrins (Brooks et al., 1994, Science 264, 569) and receptors for certain angiogenic growth factors (Hanahan, 1997, Science 277, 48). Based on these indications, it is within the scope of those skilled in the art to define an appropriate ligand moiety capable of recognizing and binding to such proteins. To illustrate, IL-2 is a suitable ligand moiety to bind to IL-2 receptor. In the case of receptors that are specific to fibrosis and inflammation, these include the TGFbeta receptors or the Adenosine receptors that are identified above and are suitable targets for invention compositions. The present application also provided methods of making any one of the compositions described herein. For example, the method of making a pharmaceutical composition comprising nanoparticles comprising a cell penetrating peptide and two or more active agents generally involve the following steps. Peptide-siRNA nanoparticles with multiple siRNA combinations are prepared by mixing amphipathic peptide and the desired individual siRNAs. Stock solutions of amphipathic peptide are prepared at 1 mg/mL (0.1-10 mg/ml range) in distilled water and sonicated for 10 min. Stock solutions of the multiple siRNA together are prepared at 100 µM (10-200uM range), total concentrations in 50 mM Tris, 0.5 mM EDTA buffer. Peptide/siRNA complexes containing multiple siRNA, are taken in equal proportions (or any desired proportion) and are formed in pure water by incubating peptide (373 µM stock solution (range 50-500uM)) with the siRNA mixture for 30 min (range 1 minute - 60 minutes) at 37C (range refrigerated to 50C) with final molar ratio of peptide and siRNA at 20:1 (range 1:1 to 100:1). Alternately, the core complex may be formed first at a peptide to siRNA ratio of about 5:1 (range 0.1:1 to 10:1) followed by a second step of incubation of the peptide with the core complex at a ratio of 20:1 (range 1:1 to 100:1). Peptide-siRNA nanoparticles can be further characterized for physicochemical properties in vitro for key parameters including particle size, surface charge, particle stability in suspension and in plasma, and siRNA integrity in plasma. In addition, these particles may be lyophilized with addition of suitable excipients such as sugars or proteins such as albumin.

### III. Methods of the present invention

The pharmaceutical compositions described herein are useful for treating fibrosis and/or inflammatory conditions. In some embodiments, the compositions are useful for reducing fibrous connective tissues in an individual having fibrosis. In some embodiments, the compositions are useful for inhibiting inflammation in an individual. These compositions can reduce fibrosis as indicated by a reduction in collagen in the relevant tissues, such as in lungs, skin, liver etc. In addition, markers of fibrosis and relevant fibrosis and inflammation related genes or gene products such as SPARC, CTGF, TGF beta receptors, TIMPs, etc. may be directly measure in relevant tissues as an indication of effectiveness. Functional endpoints in patients, in particular in patients with Idiopathic pulmonary fibrosis which are indicators of lung function such a mean 6 minute walking distance, blood oxygenation levels, etc. are also relevant indicators of the effectiveness of therapy. The compositions described herein can therefore be useful for any one or more of the following: 1) inhibiting fibrosis; 2) inhibiting inflammation; 3) reducing the amount of collagen; 4) inhibiting (such as simultaneously inhibiting) expression of two or more target genes (such as target genes described herein); 5) increasing functional walking distance; and 6) improving quality of life.

Different diseases to be treated: The present invention comprises pharmaceutical compositions to treat fibrosis or inflammatory conditions or diseases, including but not limited to: pulmonary fibrosis, idiopathic pulmonary fibrosis, progressive massive fibrosis of the lung, cystic fibrosis, mediastinal fibrosis, liver fibrosis, cirrhosis, endomyocardial fibrosis, cardiac fibrosis, old myocardial infarction, progressive kidney disease, renal fibrosis, myelofibrosis, retroperitoneal fibrosis, nephrogenic systemic fibrosis, Crohn's disease, keloids, scleroderma/systemic sclerosis, arthrofibrosis, adhesive capsulitis, fibromyalgia, peritoneal fibrosis, radiation induced fibrosis, burn induced fibrosis, trauma induced fibrosis, scarring fibrosis, and wound healing fibrosis. The invention compositions are also useful for wound healing resulting from traumatic injury such as burns or for healing of chronic wounds and reducing scarring and fibrosis.

The present invention comprises pharmaceutical compositions to treat desmoplastic cancers or cancers having a fibrotic component, including but not limited to: squamous cell carcinomas independent of their location, bilio-pancreatic carcinomas, mesothelioma, desmoplastic fibroma, desmoplastic round cell tumor, breast cancer, ovarian cancer, colorectal carcinoma and tumors of gastrointestinal tract, lung cancers, lymphomas, myelofibrosis, leukemias melanoma, brain tumors (including glioblastoma, cerebral astrocytoma, neuroblastoma, and medulloblastoma), bladder cancers, hepatocellular and urothelial tumors, and tumors of the pituitary gland.

Diseases: Idiopathic pulmonary fibrosis (IPF): IFF is a progressive and generally fatal disease characterized by scarring of the lungs that thickens the lung lining, causing an irreversible loss of lung structure and function. IPF affects 5 million people worldwide and 130,000-200,000 people in the US, with about 48,000 new cases diagnosed and 40,000 deaths each year, with medical costs estimated at $2.8 billion per 100,000 patients [2, 3]. Median survival is 2-4 years following diagnosis, and the 5-year survival is 20-40% [4, 5]. Currently, there is no known cause, no FDA approved treatments and no cure for IPF [6]. IPF patients typically are treated with anti-inflammatory drugs, including corticosteroids and cytotoxic agents, despite the fact that there is no evidence that they have any effect on long-term patient survival.

Systemic Scleroderma (SS): SS is an autoimmune disorder and a connective tissue disease that involves changes in the skin, blood vessels, muscles, and internal organs. There are an estimated 300,000 people in the US who have scleroderma, a third of whom have SS [7]. Local disease affects only skin tissues while SS affects skin and underlying tissues, blood vessels, and major organs including the heart, lungs, or kidneys. In diffuse cutaneous disease, five-year survival is 70%, and 10-year survival is 55% [8]. Currently there is no cure and no specific treatment for scleroderma. SS treatment includes immunosuppressive and anti-inflammatory drugs including corticosteroids, methotrexate, cyclophosphamide, azathioprine, and mycophenolate [9, 10].

Liver cirrhosis (LC): Liver fibrogenesis is characterized by excessive accumulation of extracellular matrix (ECM), leading to cirrhosis and complications including portal hypertension, liver failure, and hepatocellular carcinoma [11]. LC causes 800,000 deaths worldwide annually [12]. In the US, LC causes 27,000 deaths annually [13]. Established cirrhosis has a 10-year mortality of 34-66% [14]. Common causes of cirrhosis include alcohol consumption, chronic hepatitis B, C and D, obesity, toxins, bile duct diseases, and autoimmune hepatitis [15]. Currently there is no treatment available for LC, and health care costs for managing this disease are high.

Pancreatic cancer and some other cancers are known to have a fibrotic or desmoplastic stroma. However there have been no studies to specifically target the stromal components of pancreatic cancer. Peptide based nanoparticles containing siRNA targeted to CTGF, SPARC, TIMPs, TGPbeta1 or 2, MMPs and beta-catenin are of particular interest for pancreatic cancer.

Patient selection based on gene and protein expression profiling: Standard techniques of gene expression profiling, histology, immunohistochemistry, proteomic analysis, and other well accepted techniques for determining the level of DNA, mRNA, proteins and other biomarkers in patient samples can be utilized to identify the genes or proteins that are of particular interest in treating the patient. For example, if a patient is detected to have a high level expression of a particular gene or protein, then the pharmaceutical composition may be appropriately adjusted to match the particular condition of the patient. Thus, if the patient has a high level of CTGF or SPARC, the pharmaceutical composition to be administered to said patient may contain appropriate amounts of active agents to suppress CTGF or SPARC. Based on this analysis a suitable pharmaceutical composition may be selected to selectively treat or inhibit particular genes or proteins to ameliorate the pathological condition. Thus, for example, the present application in some embodiments provides a method of treating a fibrotic or inflammatory condition in an individual, comprising administering to the individual an effective amount of a pharmaceutical composition comprising one or more active agents (such as nucleic acids, including oligonucleotides for example interfering RNAs) that modulate the expression of two or more genes involved in the development or progression of fibrosis or inflammation, wherein the individual is selected based on the expression level of at least one genes involved in the development and progression of fibrosis or inflammation. In some embodiments, the active agents are selected based on the expression profile to inhibit the expression of selected target genes.

In some embodiments, there is provided a method of treating a fibrotic or inflammatory condition in an individual, comprising: 1) determining the expression level of at least one (including for example at least about any of 2, 3, 4, 5, 10, 20, 30, 40, 50, 100, 200, 300, 400, 500, or more) genes involved in the development and progression of fibrosis or inflammation, and 2) administering to the individual an effective amount of a pharmaceutical composition comprising one or more active agents (including nucleic acids, such as oligonucleotides for example interfering RNAs) that modulate the expression of two or more genes involved in the development or progression of fibrosis or inflammation. In some embodiments, the active agents are selected based on the expression profile to inhibit the expression of selected target genes.

Dosage, administration route : The pharmaceutical compositions may be administered by oral, intravenous, intraarterial, intracardiac, intracatheter, intraperitoneal, intravesical, transdermal, nasal inhalation, pulmonary delivery, intracavity, intracranial, intrathecal, subcutaneous, intradermal, intramuscular, intraocular, topical, rectal, vaginal, direct injection/administration, or local delivery with electrotransfer or microneedle injection.

Dosages of the pharmaceutical compositions of the present invention found to be suitable for treatment of human or mammalian subjects are in the range of 0.001 mg/kg - 100 mg/kg of the active agent. More preferred dosage ranges are 0.1-20 mg/kg and more preferably in the range of 0.5-10 mg/kg, e.g. 0.1-0.5, 0.5-1. The schedule of administration to the subject may range from a single administration that constitutes the entire treatment to daily administration. More preferably, the administration is once every 3-30 days and most preferably once every 4-7 days.

### IV. Kits, compositions, reagents, and article of manufacture

Also provided herein are kits, reagents, and articles of manufacture useful for the methods described herein. Such kits may contain vials containing the carrier molecules separately from vials containing the active agents. At the time of patient treatment, it is first determined what particular pathology is to be treated based on for example, gene expression analysis or proteomic or histological analysis of patient samples. Having obtained those results, the carrier molecules are mixed with the appropriate active agent molecules to result in complexes or nanoparticles that can be administered to the patient for an effective treatment. Thus, in some embodiments, there is provided a kit comprising: 1) two or more active agents (such as nucleic acids, for example oligonucleotides); 2) a cell-penetrating peptide. In some embodiments, the kit further comprises agents for determining gene expression profiles. In some embodiment, the kit further comprises a pharmaceutically acceptable carrier.

The kits described herein may further comprise instructions for using the components of the kit to practice the subject methods (for example instructions for making the pharmaceutical compositions described herein and/or for use of the pharmaceutical compositions). The instructions for practicing the subject methods are generally recorded on a suitable recording medium. For example, the instructions may be printed on a substrate, such as paper or plastic, etc. As such, the instructions may be present in the kits as a package insert, in the labeling of the container of the kits or components thereof (i.e., associated with the packaging or sub packaging) etc. In some embodiments, the instructions are present as an electronic storage data file present on a suitable computer readable storage medium, e.g., CD-ROM, diskette, etc. In yet other embodiments, the actual instructions are not present in the kit, but means for obtaining the instructions from a remote source, e.g., via the internet, are provided. An example of this embodiment is a kit that includes a web address where the instructions can be viewed and/or from which the instructions can be downloaded. As with the instructions, this means for obtaining the instructions is recorded on a suitable substrate.

The various components of the kit may be in separate containers, where the containers may be contained within a single housing, e.g., a box.

Further provided herein are methods of making any of the articles of manufacture described herein.

The invention is described in detail below and comprises :
A method of treating a fibrotic or inflammatory condition in an individual, comprising administering to the individual an effective amount of a pharmaceutical composition comprising one or more nucleic acids that modulate the expression of two or more genes involved in the development or progression of fibrosis or inflammation.

In some embodiments, the two or more genes are selected from the group consisting of CTGF(CCN2), TGFbetai, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, smoothened, GLI1, GLI2, and Patched-1. Yet another gene of interest for the present invention is HIF-1alpha.

In some embodiments, there is provided a method of treating a fibrotic or inflammatory condition in an individual, comprising administering to the individual an effective amount of a pharmaceutical composition comprising one or more active agents that modulate the activity or expression of two or more genes involved in the development and progression of fibrosis or inflammation, selected from, CTGF(CCN2), TGFbeta1, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STATS, smoothened, GLI1, GLI2, and Patched-1. Yet another gene of interest for the present invention is HIF-1alpha.

In some embodiments, according to methods described above, the one or more active agents are nucleic acids.

In some embodiments, according to methods described above, at least one of the nucleic acids is a single stranded oligonucleotide.

In some embodiments, according to methods described above, at least one of the nucleic acids is a double stranded oligonucleotide.

7 In some embodiments, according to methods described above, at least one of the nucleic acids is selected from the group consisting of antisense oligonucleotide, RNAi, shRNA, siRNA, miRNA, or plasmid DNA.

In some embodiments, according to methods described above, at least one of the nucleic acids is RNAi.

In some embodiments, according to methods described above, at least one of the nucleic acids is siRNA.

In some embodiments, according to methods described above, the at least one of the nucleic acids is shRNA.

In some embodiments, according to methods described above, at least one of the nucleic acids is about 10 to about 50 nucleotides long.

In some embodiments, according to methods described above, the composition comprises two or more nucleic acids.

In some embodiments, according to methods described above, the two or more nucleic acids are of the same kind.

In some embodiments, according to methods described above, the two or more nucleic acids are of different kinds.

In some embodiments, according to methods described above, the composition comprises two nucleic acids, and wherein the molar ratio of the two nucleic acids is about 0.1:1 to about 10:1.

In some embodiments, according to methods described above, the two or more nucleic acids in the pharmaceutical composition are in equal molar proportions.

In some embodiments, according to methods described above, the nucleic acids are associated with a carrier molecule.

In some embodiments, according to methods described above, the nucleic acids are covalently associated with the carrier molecule.

In some embodiments, according to methods described above, the nucleic acids are non-covalently associated with the carrier molecule.

In some embodiments, according to methods described above, the carrier molecule is selected from the group consisting of a peptide, a protein, an antibody, a lipid, a phospholipid, a polymer, an aptamer, a nanoparticle, a liposome, a dendrimer, a polymerosome, a viral vector, and a micelle.

In some embodiments, according to methods described above, the carrier molecule is a peptide.

In some embodiments, according to methods described above, the peptide is a cell-penetrating peptide.

In some embodiments, according to methods described above, the cell-penetrating peptide is selected from the group consisting of a PTD-based peptide, an amphipathic peptide, a poly-arginine-based peptide, a MPG peptide, a CADY peptide, Pep-1 peptide or a Pep-2 peptide,.

In some embodiments, according to methods described above, the nucleic acids and the cell-penetrating peptide are present in a complex.

In some embodiments, according to methods described above, the composition comprises nanoparticles comprising the complexes of the nucleic acids and the cell-penetrating peptide.

In some embodiments, according to methods described above, the average size of the nanoparticles is between 50 and 400 nm.

In some embodiments, according to methods described above, the molar ratio of the cell-penetrating peptide and the nucleic acids in the composition is about 100:1 to about 1:50.

In some embodiments, according to methods described above, the active agents modulate the activity or expression of two or more genes selected from: SPARC, CTGF, TGFbetai, TGFbeta receptor 1, TGFbeta receptor 2, TGFbeta receptor 3, beta-catenin, Adenosine receptor A2A, Adenosine receptor A2B, TIMPs.

In some embodiments, according to methods described above, the active agents modulate the activity of SPARC and TGFbeta1.

In some embodiments, according to methods described above, the fibrotic or inflammatory condition is selected from the group consisting of pulmonary fibrosis, idiopathic pulmonary fibrosis, progressive massive fibrosis of the lung, cystic fibrosis, mediastinal fibrosis, liver fibrosis, cirrhosis, endomyocardial fibrosis, cardiac fibrosis, old myocardial infarction, progressive kidney disease, renal fibrosis, myelofibrosis, retroperitoneal fibrosis, nephrogenic systemic fibrosis, Crohn's disease, keloids, scleroderma/systemic sclerosis, arthrofibrosis, adhesive capsulitis, fibromyalgia, peritoneal fibrosis, radiation induced fibrosis, burn induced fibrosis, trauma induced fibrosis, scarring fibrosis, and wound healing fibrosis.

In some embodiments, according to methods described above, the fibrotic or inflammatory conditions is a desmoplastic cancer, wherein the desmoplastic cancer is selected from the group consisting of: squamous cell carcinoma independent of their location, bilio-pancreatic carcinoma, mesothelioma, desmoplastic fibroma, desmoplastic round cell tumor, breast cancer, ovarian cancer, colorectal carcinoma and tumor of gastrointestinal tract, lung cancer, lymphoma, myelofibrosis, leukemia, melanoma, brain tumor (including glioblastoma, cerebral astrocytoma, neuroblastoma, and medulloblastoma), bladder cancer, hepatocellular and urothelial tumor, and tumor of the pituitary gland.

In some embodiments, according to methods described above, said pharmaceutical composition is administered by oral, intravenous, intraarterial, intracardiac, intracatheter, intraperitoneal, intravesical, transdermal, nasal inhalation, pulmonary delivery, intracavity, intracranial, intrathecal, subcutaneous, intradermal, intramuscular, intraocular, topical, rectal, vaginal, direct injection/administration, or local delivery with electrotransfer or microneedle injection.

In some embodiments, according to methods described above, the individual is selected based on the expression level of at least one genes involved in the development and progression of fibrosis or inflammation.

In some embodiments, according to methods described above, the invention requires further determining the expression level of at least one gene prior to the administration of the pharmaceutical composition.

In some embodiments, the invention comprises a pharmaceutical composition comprising two or more active agents that modulate the expression of two or more genes involved in the development or progression of fibrosis or inflammation.

In some embodiments, the invention requires thai the two or more genes are selected from the group consisting of CTGF(CCN2), TGFbetai, TGFbeta receptor 1, TGFbeta receptor 2, TGFbeta receptor 3, beta-catenin, SPARC, VEGF, Angiotensin 11, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, , SOX9, arrestin, PDCD4, PAI-1, NF-kB, and PARP-1 GAD65, sGAD65, BAX, p53 PTEN, STAT5, smoothened, GLI1, GLI2, and Patched-1. Yet another gene of interest for the present invention is HIF-1alpha

In some embodiments, the invention requires that the two or more active agents are of the same kind.

In some embodiments, the invention requires that the two or more active agents are of different kinds.

In some embodiments, the invention requires that the composition comprises two active agents, and wherein the molar ratio of the two active agents is about 0.1:1 to about 10:1.

In some embodiments, the invention requires that the two or more active agents in the pharmaceutical composition are in equal molar proportions.

In some embodiments, the invention requires that the two or more active agents are nucleic acids.

In some embodiments, the invention requires that at least one of the nucleic acids is a single stranded oligonucleotide.

In some embodiments, the invention requires that at least one of the nucleic acids is a double stranded oligonucleotide.

In some embodiments, the invention requires that at least one of the nucleic acids is selected from the group consisting of antisense oligonucleotide, RNAi, shRNA, siRNA, miRNA, or plasmid DNA.

In some embodiments, the invention requires that at least one of the nucleic acids is RNAi.

In some embodiments, the invention requires that at least one of the nucleic acids is siRNA.

In some embodiments, the invention requires that at least one of the nucleic acids is shRNA.

In some embodiments, the invention requires that at least one of the nucleic acids is about 10 to about 50 nucleotides long.

In some embodiments, the invention requires that the nucleic acids are associated with a carrier molecule.

In some embodiments, the invention requires that the nucleic acids are covalently associated with the carrier molecule.

In some embodiments, the invention requires that the nucleic acids are non-covalently associated with the carrier molecule.

In some embodiments, the invention requires that the carrier molecule is selected from the group consisting of a peptide, a protein, an antibody, a lipid, a phospholipid, a polymer, an aptamer, a nanoparticle, a liposome, a dendrimer, a polymerosome, a viral vector, and a micelle.

In some embodiments, the invention requires that the carrier molecule is a peptide.

In some embodiments, the invention requires that the peptide is a cell-penetrating peptide.

In some embodiments, the invention requires that the cell-penetrating peptide is selected from the group consisting of a PTD-based peptide, an amphipathic peptide, a poly-arginine-based peptide, a MPG peptide, a CADY peptide, Pep-1 peptide, or a Pep-2 peptide.

In some embodiments, the invention requires that the nucleic acids and the cell-penetrating peptide are present in a complex.

In some embodiments, the invention requires that the composition comprises nanoparticles comprising the complexes of the nucleic acids and the cell-penetrating peptide.

In some embodiments, the invention requires that the average size of the nanoparticles is between 50 and 400 nm.

In some embodiments, the invention requires that the molar ratio of the cell-penetrating peptide and the nucleic acids in the composition is about 100:1 to about 1:50.

### EXAMPLES

The following are examples of methods and compositions of the invention. It is understood that various other embodiments may be practiced, given the general description provided above.

### Example 1a:

This example provides information on preparation of peptide-siRNA complexes. Preparation and characterization of peptide-siRNA nanoparticles: The siRNA sequences will be designed to target 8 selected mouse genes: TGFbeta receptors 1 and 2, CTGF, adenosine receptors A2A and A2B, SPARC, and TIMP-1 and -3. Peptide-siRNA nanoparticles are prepared by mixing amphipathic peptide and individual siRNAs targeting fibresis-related genes. Stock solutions of amphipathic peptide are prepared at 1 mg/mL in distilled water and sonicated for 10 min. Stock solutions of siRNA are prepared at 100 µM concentrations in 50 mM Tris, 0.5 mM EDTA buffer. Peptide/siRNA complexes are formed in pure water by incubating peptide (373 µM stock solution) with siRNA (100 µM stock solution) for 30 min at 37C with final molar ratio of peptide and siRNA at 20:1. Peptide-siRNA nanoparticles will be characterized for physicochemical properties in vitro for key parameters including particle size, surface charge, particle stability in suspension and in plasma, and siRNA integrity in plasma.

### Example 1b:

This example provides information on preparation of peptide-multiple siRNA complexes. Preparation and characterization of peptide-siRNA nanoparticles: The siRNA sequences will be designed to target 8 selected mouse genes: TGFbeta receptors 1 and 2, CTGF, adenosine receptors A2A and A2B, SPARC, and TIMP-1 and -3. Peptide-siRNA nanoparticles with multiple siRNA combinations are prepared by mixing amphipathic peptide and the desired individual siRNAs targeting fibrosis-related genes. Stock solutions of amphipathic peptide are prepared at 1 mg/mL in distilled water and sonicated for 10 min. Stock solutions of the multiple siRNA together are prepared at 100 µM total concentrations in 50 mM Tris, 0.5 mM EDTA buffer. Peptide/siRNA complexes containing multiple siRNA, e.g., siRNA for SPARC, TGFbeta receptor 1 and adenosine receptors A2A are taken in equal proportions and are formed in pure water by incubating peptide (373 µM stock solution) with the siRNA mixture (100 µM stock solution) for 30 min at 37C with final molar ratio of peptide and siRNA at 20:1. Alternately, the core complex may be formed first at a peptide to siRNA ratio of about 5:1 followed by a second step of incubation of the peptide with the core complex at a ratio of 20:1. Peptide-siRNA nanoparticles will be characterized for physicochemical properties in vitro for key parameters including particle size, surface charge, particle stability in suspension and in plasma, and siRNA integrity in plasma.

### Example 2:

This example provides information to show that SPARC and CTGF siRNA reduce expression of collagen type 1 in skin fibroblasts of CTGF transgenic mice. Transgenic mice that over-express connective tissue growth factor (CTGF) in fibroblasts under the control of an enhancer/promoter element of the Colla2 gene (Colla2-CTGF) recapitulate multiorgan fibrosis similar to fibrosis observed in Scleroderma (SSc). In this study the regulation of Sparc and Ctgf siRNAs on the expression of several extracellular matrix components in the fibroblasts derived from Colla2-CTGF transgenic mice was investigated. Three fibroblast lines were obtained from each of wide type C57BL/6 and CTGF transgenic C57BL/6, and were transfected with either Sparc siRNA or Ctgf siRNA. Real-time quantitative RT-PCR and Western blotting were used to examine the transcription and protein levels of type I collagen, CTGF and SPARC. Student's t-tests were used to determine the significance of the results. The results showed that Colla2 and Ctgf increased expression at both transcriptional and translational levels in the fibroblasts from the Colla2-CTGF transgenic mice compared with those in the fibroblasts from their normal wild-type littermate. The treatment with Sparc siRNA or Ctgf siRNA attenuated the mRNA and/or protein expression of the Colla2, Ctgf and Sparc in these fibroblasts. Sparc and Ctgf siRNAs also showed a reciprocal inhibition at transcript levels. Therefore, the results indicated that both SPARC and CTGF independently appeared to be involved in the same biological pathway, and they have the potential to serve as a therapeutic target for fibrotic diseases such as SSc.

### Example 3:

This example provides information to show that SPARC and CTGF siRNA reduce bleomycin induced skin and lung fibrosis in mice. SPARC is a matricellular protein, which, along with other extracellular matrix components including collagens, is commonly over-expressed in fibrotic diseases. The purpose of this study was to examine whether inhibition of SPARC can regulate collagen expression in vitro and in vivo, and subsequently attenuate fibrotic stimulation by bleomycin in mouse skin and lungs. In in vitro studies, skin fibroblasts obtained from a Tgfbr1 knock-in mouse (TBR1CA: Cre-ER) were transfected with SPARC siRNA. Gene and protein expressions of the Colla2 and the Ctgf were examined by real-time RT-PCR and Western blotting, respectively. In in vivo studies, C57BL/6 mice were induced for skin and lung fibrosis by bleomycin and followed by SPARC siRNA treatment through subcutaneous injection and intratracheal instillation, respectively. The pathological changes of skin and lungs were assessed by hematoxylin and eosin and Masson's trichrome stains. The expression changes of collagen in the tissues were assessed by real-time RT-PCR and non-crosslinked fibrillar collagen content assays. SPARC siRNA significantly reduced gene and protein expression of collagen type 1 in fibroblasts obtained from the TBR1CA; Cre-ER mouse that was induced for constitutively active TGF-beta receptor I. Skin and lung fibrosis induced by bleomycin was markedly reduced by treatment with SPARC siRNA. The anti-flbrotic effect of SPARC siRNA in vivo was accompanied by an inhibition of Ctgf expression in these same tissues. Specific inhibition of SPARC effectively reduced fibrotic changes in vitro and in vivo. SPARC inhibition may represent a potential therapeutic approach to fibrotic diseases

### Example 4:

This example provides information to show the Vitro Evaluation of Peptide-siRNA Nanoparticles.

The peptide-siRNA nanoparticles carrying individual siRNAs against fibrogenic genes and their combinations will be evaluated in vitro in cell culture of 3 different fibrosis models for the efficacy of siRNA delivery into the cells, target down regulation, and the inhibition of collagen deposition. The effect of different siRNAs will be compared to identify the interaction between multiple fibrogenic genes and pathways and rationally select optimal combinations for in vivo testing against fibrosis mouse models. In vitro siRNA delivery by peptide-siRNA nanoparticles in cell cultures of 3 fibroblast models:
- Cell culture: Three established in vitro cell lines of fibrosis are selected: CTGF overexpressing mouse fibroblasts isolated from CTGF transgenic mice [21], TGFbeta receptor 1 overexpressing mouse skin fibroblasts isolated with TGFbetaR1 knock-in mice [29], and mouse skin fibroblasts isolated from ADA-null mice [39].
- In vitro siRNA delivery: Nanoparticles containing different siRNAs and their combinations and scrambled siRNA are incubated with mouse fibroblasts for 30 minutes followed by fresh DMEM/10%FCS.

Collection and Processing of Samples: Seventy-two hours following siRNA delivery, cells are harvested, followed by mRNA and protein purification.

Analysis: The mRNA and protein levels of target genes and collagen I are analyzed by real time RT-PCR and western blot, respectively.

Outcomes: Levels of target and collagen I mRNA and proteins will be compared between control and the treated groups to determine the extent of gene knockdown and the effect on collagen deposition. Effective down regulation of key fibrogenic genes can abolish the collagen deposition shown in the in vitro fibrosis models. The effect of individual siRNA and combinations on different fibrogenic genes will be analyzed to determine interactions between multiple fibrotic genes and signaling pathways and to identify potential synergistic interactions.

### Example 5:

This example provides information to show the In Vivo Screening and Dose Ranging Study of Peptide-siRNA Nanoparticles

In vivo screening and dose ranging study will be conducted in bleomycin pulmonary and adenosine deaminase deficient (ADA-null) mouse models to identify optimal siRNA combinations and dose. Different mouse fibrosis models have distinct molecular pathology for disease progression. Therefore, 2 independent models of fibrosis are employed for in vivo testing of peptide-siRNA nanoparticles selected through in vitro screening in Example 4. Pulmonary fibrosis model caused by intratracheal (IT) instillation of bleomycin was reported before [29]. ADA-null mice lacking adenosine deaminase accumulate high levels of adenosine, which results in severe diffuse dermal fibrosis and premature deaths 3 weeks after birth from pulmonary inflammation and fibrosis [40]. Testing of selected peptide-siRNA nanoparticles at different dose levels will determine a safe and effective dose for target gene down regulation, as well as provide preliminary indication on the anti-fibrogenic efficacy in vivo. The siRNA nanoparticles will be administered both intravenously (IV) and IT in the bleomycin pulmonary fibrosis model to compare the safety and efficacy of systemic and local delivery. The ADA-null mice will be administered IV to test the ability for systemic therapy to control a diffused systemic fibrotic disease. The key promoters of fibrosis in different models can be identified and used to rationally design the most optimal siRNA combinations for in vivo efficacy testing in Example 5.

Mouse fibrosis models: Bleomycin-induced pulmonary fibrosis model will be generated following established method by one-time IT instillation of C57BL/6 mice with 3.5 units/kg bleomycin dissolved in saline [29]. ADA-deficient mice were generated as described previously [39].

Administration of siRNA nanoparticles in vivo:
- Bleomycin pulmonary fibrosis model: On Days 2, 5, 12 after bleomycin treatment, each siRNA nanoparticle group is administered either IV or IT at 3 different dose levels of siRNA: 0.15 mg/kg (3 ug/mouse), 0.5 mg/kg (10 ug/mouse), and 1.5 mg/kg (30 ug/mouse). Animal groups (n = 3/group) are: 1) control (C57BL/6 mice with one IT instillation of saline); 2) Bleomycin (C57BL/6 mice with one IT instillation of bleomycin); 3) Scrambled IV (bleomycin mice treated IV with 3 doses of nanoparticles carrying scrambled siRNA); 4) Scrambled IT; 5-14) siRNA IV (estimated 4 individual siRNAs and 6 different siRNA combinations, total of 10 siRNA treatment groups treated IV at 3 dose levels - 1 animal/level); 15-24) siRNA IT. Total animal number: 3 x 24 group = 72 C57BL/6 mice.
- ADA-null mice: On Days 2, 5, 12 after birth, the mice are treated IV with different siRNA nanoparticles at 3 dose levels: 0.15 mg/kg, 0.5 mg/kg, and 1.5 mg/kg. Animal groups with ADA-null mice (n = 3/group) are treated with: 1) Saline; 2) Scrambled siRNA: 3-12) siRNA (10 siRNA nanoparticle groups at 3 dose levels - 1 animal/level). Total animal number: 3 x 12 groups = 36 ADA-null mice.

Collection and Processing of Samples: For bleomycin-treated mice, all mice will be sacrificed on Day 23, and the lung samples are collected. The left lungs are fixed by 4% formalin and used for further histological analysis. The right lungs are minced to small pieces and divided for RNA extraction and collagen content analysis. For ADA-null mice, all mice will be sacrificed on Day 23 after birth, and the lung, skin and liver are collected and analyzed for histology, RNA and collagen protein levels.

Analysis: Samples harvested from mice treated with scrambled or test siRNA are analyzed for target gene down regulation by real time RT-PCR for RNA levels and by western blot for protein expression. Collagen is visualized using H&E and Masson's trichrome staining, and measured with Sircol colorimetric assay (Biocolor, Belfast, UK) following method previously described [29].

Outcomes: The comparison of mRNA and protein expression profiles between control, bleomycin-treated, and siRNA treated C57BL/6 mice will reveal key fibrotic genes and their interactions. Treatment with siRNA nanoparticles by IV or IT will significantly down regulate the mRNA and protein levels of target genes and potentially other fibrotic genes in a dose-dependent manner. Robust knockdown of key fibrotic genes will be correlated with significant reduction of collagen deposition, as demonstrated both by histology and collagen content measurement. Survival of ADA-null mice may potentially be prolonged by siRNA nanoparticle treatment. The 3 most effective siRNA nanoparticle combinations will be selected for expanded efficacy testing in Example 5.

### Example 6:

This example provides information to show the In Vivo Evaluation of Safety and Anti-fibrosis Efficacy of Selected siRNA Combinations

The in vivo safety and anti-fibrosis efficacy of 3 selected siRNA combinations will be evaluated in bleomycin pulmonary fibrosis and ADA-null mouse models. Preliminary study in Example 5 will suggest the 3 most effective siRNA nanoparticle combinations and a safe dose for in vivo delivery via IV and IT routes. The study in this aim seeks to demonstrate the safety and efficacy of the selected siRNA combinations with a longer repeated treatment and narrow to 1 optimal siRNA combination for further development into a potential therapeutic agent.

Mouse fibrosis models: Bleomycin-induced pulmonary fibrosis model and ADA-deficient mice are described in Example 5.

Administration of siRNA nanoparticles in vivo:
- Bleomycin pulmonary fibrosis model: On Days 2, 5, 12, 19, 26 after bleomycin treatment, mice will be treated with the selected siRNA combinations at dose determined in Example 5. Mice are monitored for weight loss and signs of toxicity following siRNA treatment. Animal groups (n = 8/group) are: 1) control; 2) Bleomycin; 3) Scrambled IV; 4) Scrambled IT; 5-7) siRNA IV; 8-10) siRNA IT. Total animal number: 8 x 10 group = 80 C57BL/6 mice.
- ADA-null mice: On Days 2, 5, 12 after birth, the mice are treated IV with the selected siRNA combinations at dose determined in Example 4. Mice are monitored for weight loss and signs of toxicity following siRNA treatment. Animal groups with ADA-null mice (n = 8/group) are treated with: 1) Saline; 2) Scrambled siRNA; 3-5) siRNA. Total animal number: 8 x 5 groups = 40 ADA-null mice.

Collection and Processing of Samples: Sample collection and processing will be at Day 36 follow method outlined in the Example 4.

Analysis: Samples analysis will follow method outlined in Example 5.

Outcomes: The results from this study will in general agree with preliminary data from Example 4. The knockdown of target and other fibrotic genes will correlate with anti-fibrotic efficacy as shown by decrease in collagen deposition in fibrosis lesions as shown by histological analysis and collagen content measurement. The siRNA combination most effective in decreasing levels of key fibrotic genes and reducing fibrosis in the 2 mouse fibrosis models will be selected for further development into a therapeutic agent for human fibrotic diseases.

### Example 7:

This example provides information to show the Increased stability of Peptide-plasmid DNA Nanoparticles

The peptide-plasmid nanoparticles were prepared by mixing amphipathic peptide and plasmid. Stock solutions of amphipathic peptide are prepared at 1 mg/mL in distilled water and sonicated for 10 min. Stock solutions of plasmid are prepared at 100 µM concentrations in 50 mM Tris, 0.5 mM EDTA buffer. Peptide/plasmid complexes or nanoparticles are formed in pure water by incubating peptide (373 µM stock solution) with plasmid (100 µM stock solution) for 30 min at 37C with final molar ratio of peptide and plasmid at 10:1 or 20:1. The plasmid alone and the peptide/plasmid complex are stored in PBS for 1 week at 40C to test the stability of the plasmid. The percentage of supercoiled DNA is measured after 1 week using agarose gel electrophoresis using standard techniques (see for example - Pillai VB, Hellerstein M, Yu T, Amara RR, Robinson HL. Comparative studies on in vitro expression and in vivo immunogenicity of supercoiled and open circular forms of plasmid DNA vaccines. Vaccine. 2008 Feb 20;26(8):1136-41). It takes approximately 7 days for 95% supercoiled DNA in PBS to reach 80% supercoil at 40C. In the case of the peptide-plasmid complex, the level of supercoil is maintained > 90% at 1 week.

### Example 8:

This example provides information to show the treatment of a cancer with fibrosis and inflammation (pancreatic cancer) with selected siRNA combinations

Pancreatic cancer and some other cancers are known to have a fibrotic or desmoplastic stroma. However there have been no studies to specifically target the stromal components of pancreatic cancer. Peptide based nanoparticles containing siRNA targeted to CTGF, SPARC, TIMPs, TGFbeta1 or 2, MMPs and beta-catenin were prepared as described above and administered to athymic mice with direct from patient pancreatic xenografts or used in genetically engineered mice that spontaneously develop pancreatic cancer. Intravenous administration was initiated once tumors were detectable. Animals were treated every 4 days for 5 treatments along with a control group. Tumor size was monitored throughout the course of the study and tumor histology was determined after 5 treatments. A significant reduction in the stromal components, in particular collagen was observed after treatment.

LIST OF REFERENCES which are incorporated herein in their entirety for all uses:
A publication by Cronstein [2011, http://f1000.com/reports/b/3/21; http://f1000.com/reports/b/3/21/pdf] highlights the importance of Adenosine receptors and fibrosis is incorporated herein by reference in its entirety.
Attenuation of fibrosis in vitro and in vivo with SPARC siRNA:. A publication by Wang et al (Arthritis Res Ther. 2010;12(2):R60. Epub 2010 Apr 1.) highlights the importance of SPARC and fibrosis and is incorporated by reference herein in its entirety.
Attenuation of expression of extracellular matrix genes with siRNAs to Spare and Ctgf in skin fibroblasts of CTGF transgenic mice. A publication by Wang et al. (Int J Immunopathol Pharmacol. 2011 Jul-Sep;24(3):595-601) highlights the importance of SPARC and fibrosis and is incorporated by reference herein in its entirety.
SPARC Suppresses Apoptosis of Idiopathic Pulmonary Fibrosis Fibroblasts through Constitutive Activation of beta-Catenin : A publication by Chang et al [The Journal of Biological Chemistry, Vol. 285, NO. 11, pp. 8196-8206, March 12, 2010] highlights the relevance of SPARC in pulmonary fibrosis and is incorporated by reference herein in its entirety.
Secreted Protein Acidic And Rich In Cysteine (sparc) Expression Is Regulated By Selectively Tgf-Beta Via Pi3k Signaling: A publication by S. Shibata1, J. Ishiyama1, K. Hagihara1, K. Murakami (Am J Respir Crit Care Med 183;2011:A3478) is incorporated by reference herein in its entirety.
Adenovirus-mediated inhibition of SPARC attenuates liver fibrosis in rats: Camino AM, Atorrasagasti C, Maccio D, Prada F, Salvatierra E, Rizzo M, Alaniz L, Aquino JB, Podhajcer OL, Silva M, Mazzolini G. [J Gene Med. 2008 Sep;10(9):993-1004.] the contents of this publication are incorporated herein in its entirety.
TGF-beta and fibrosis in different organs - molecular pathway imprints: Pohlers D, Brenmoehl J, Löffler I, Müller CK, Leipner C, Schultze-Mosgau S, Stallmach A, Kinne RW, Wolf G. [Biochim Biophys Acta. 2009 Aug;1792(8):746-56. Epub 2009 Jun 17] the contents of this publication are incorporated herein in its entirety.
TGF-beta1 Gene Silencing for Treating Liver Fibrosis: (Kun Cheng, Ningning Yang and Ram I. Mahato: Mol. Pharmaceutics, 2009, 6 (3), pp 772-779) - the contents of this publication are incorporated herein in its entirety.
Fibrotic disease: A pair of novel targets: Charlotte Harrison [Nature Reviews Drug Discovery 8, 773 (October 2009) | doi:10.1038/nrd3005] the contents of this publication are incorporated herein in its entirety.
Potential Therapeutic Targets for Cardiac Fibrosis: TGFbeta, Angiotensin, Endothelin, CCN2 (CTGF), and PDGF, Partners in Fibroblast Activation. Andrew Leask [Circulation Research. 2010: 106: 1675-1680] the contents of this publication are incorporated herein in its entirety.
Small interfering RNAs (siRNAs) targeting TGF-beta1 mRNA suppress asbestos-induced expression of TGF-beta1 and CTGF in fibroblasts: (Lai TC, Pociask DA, Ferris M, Nguyen HT, Miller CA, Brody A, Sullivan D; J Environ Pathol Toxicol Oncol. 2009 . 28(2):109-19) the contents of this publication are incorporated herein in its entirety.
Inhibition of TGF-beta receptor I by siRNA suppresses the motility and invasiveness of T24 bladder cancer cells via modulation of integrins and matrix metalloproteinase: Li Y, Yang K, Mao Q, Zheng X, Kong D, Xie L ; Int Urol Nephrol. 2010 Jun;42(2):315-23. Epub 2009 Aug 8. the contents of this publication are incorporated herein in its entirety
Modulation of collagen synthesis in keloid fibroblasts by silencing Smad2 with siRNA: Plast Reconstr Surg. 2006 Nov;118(6):1328-37. Gao Z, Wang Z, Shi Y , Lin Z , Jiang H , Hou T , Wang Q , Yuan X , Zhao Y, Wu H , Jin Y. the contents of this publication are incorporated herein in its entirety.
US patent 8,067,389 describes Silencing of TGFbeta. type II receptor expression by specific siRNA sequences, the contents of which are incorporated herein in their entirety.
US patent 8,003,621 discloses compositions that can include a cationic polymeric carriers, targeting agent, and therapeutic agent having a therapeutic activity such as inhibiting fibrosis within a target organ or tissue or inhibiting the growth of a cancer cell, the contents of which are incorporated herein in their entirety.

### [numbered references below correspond to numbers in text]

1. Wynn TA. Cellular and molecular mechanisms of fibrosis. J Pathol. 2008 Jan;214(2): 199-210.
2. Raghu G, Weycker D, Edelsberg J, Bradford WZ, Oster G. Incidence and prevalence of idiopathic pulmonary fibrosis. Am J Respir Crit Care Med. 2006 Oct 1;174(7):810-6.
3. Buck M, Chojkier M. C/EBPbeta-Thr217 phosphorylation signaling contributes to the development of lung injury and fibrosis in mice. PLoS One 2011;6(10):e25497.
4. American Thoracic Society. Idiopathic pulmonary fibrosis: diagnosis and treatment. International consensus statement. American Thoracic Society (ATS), and the European Respiratory Society (ERS). Am J Respir Crit Care Med. 2000 Feb;161(2 Pt 1):646-64.
5. Olson AL, Swigris JJ, Lezotte DC, Norris JM, Wilson CG, Brown KK. Mortality from pulmonary fibrosis increased in the United States from 1992 to 2003. Am J Respir Crit Care Med. 2007 Aug 1;176(3):277-84.
6. du Bois RM. Strategies for treating idiopathic pulmonary fibrosis. Nat Rev Drug Discov. 2010 Feb;9(2):129-40.
7. Scleroderma Foundation. What is scleroderma? ; 2012 [updated 2012; cited]; Available from: http://www.scleroderma.org/medical/overview.shtm.
8. Gilliand B. Systemic sclerosis (scleroderma) and related disorders. Harrison's Principles of Internal Medicine, 16th Edition: McGraw-Hill Medical Publishing; 2004.
9. Sapadin AN, Fleischmajer R. Treatment of scleroderma. Arch Dermatol. 2002 Jan;138(1):99-105.
10. Leighton C. Drug treatment of scleroderma. Drugs. 2001;61(3):419-27.
11. Liu X, Hu H, Yin JQ. Therapeutic strategies against TGF-beta signaling pathway in hepatic fibrosis. Liver Int. 2006 Feb;26(1):8-22.
12. World Health Organization, editor. The World Health Report 2002: Reducing Risks, Promoting Healthy Life; 2002; Geneva.
13. Minino AM, Heron MP, Murphy SL, Kochanek KD. Deaths: final data for 2004. Natl Vital Stat Rep. 2007 Aug 21;55(19):1-119.
14. Sorensen HT, Thulstrup AM, Mellemkjar L, Jepsen P, Christensen E, Olsen JH, Vilstrup H. Long-term survival and cause-specific mortality in patients with cirrhosis of the liver: a nationwide cohort study in Denmark. J Clin Epidemiol. 2003 Jan;56(1):88-93.
15. National Digestive Diseases Information Clearinghouse (NDDIC). Cirrhosis. 2008 [updated 2008; cited]; Available from: http://digestive.niddk.nih.gov/ddiseases/pubs/cirrhosis/.
16. Pohlers D, Brenmoehl J, Loffler I, Muller CK, Leipner C, Schultze-Mosgau S, Stallmach A, Kinne RW, Wolf G. TGF-beta and fibrosis in different organs - molecular pathway imprints. Biochim Biophys Acta. 2009 Aug;1792(8):746-56.
17. Hecker L, Vittal R, Jones T, Jagirdar R, Luckhardt TR, Horowitz JC, Pennathur S, Martinez FJ, Thannickal VJ. NADPH oxidase-4 mediates myofibroblast activation and fibrogenic responses to lung injury. Nat Med. 2009 Sep;15(9):1077-81.
18. Ihn H. Pathogenesis of fibrosis: role of TGF-beta and CTGF. Current opinion in rheumatology. 2002 Nov;14(6):681-5.
19. George J, Tsutsumi M. siRNA-mediated knockdown of connective tissue growth factor prevents N-nitrosodimethylamine-induced hepatic fibrosis in rats. Gene Ther. 2007 May;14(10):790-803.
20. Shibata S, Ishiyama J, Hagihara K, Murakami K. Secreted Protein Acidic And Rich In Cysteine (sparc) Expression Is Regulated By Selectively Tgf-Beta Via Pi3k Signaling. Am J Respir Crit Care Med.2011;183:A3478.
21. Wang JC, Sonnylal S, Arnett FC, De Crombrugghe B, Zhou X. Attenuation of expression of extracellular matrix genes with siRNAs to Sparc and Ctgf in skin fibroblasts of CTGF transgenic mice. Int J Immunopathol Pharmacol. 2011 Jul-Sep;24(3):595-601.
22. Rachfal AW, Brigstock DR. Connective tissue growth factor (CTGF/CCN2) in hepatic fibrosis. Hepatol Res. 2003 May;26(1):1-9.
23. Wang J, Lai S, Sonnylal S, Arnett F, de Crombrugghe B, Zhou X. Application Of Sparc And Ctgf siRNA In Fibrotic Murine Models Of Scleroderma (SSc) In Vitro And In Vivo. American College of Rheumatology 2008 Annual Scientific Meeting; 2008; San Francisco, California. 2008.
24. Cronstein BN. Adenosine receptors and fibrosis: a translational review. F1000 Biol Rep. 2011;3:21.
25. Zhou Y, Murthy JN, Zeng D, Belardinelli L, Blackburn MR. Alterations in adenosine metabolism and signaling in patients with chronic obstructive pulmonary disease and idiopathic pulmonary fibrosis. PLoS One. 2010;5(2):e9224.
26. Chan ES, Montesinos MC, Fernandez P, Desai A, Delano DL, Yee H, Reiss AB, Pillinger MH, Chen JF, Schwarzschild MA, Friedman SL, Cronstein BN. Adenosine A(2A) receptors play a role in the pathogenesis of hepatic cirrhosis. Br J Pharmacol. 2006 Aug; 148(8):1144-55.
27. Che J, Chan ES, Cronstein BN. Adenosine A2A receptor occupancy stimulates collagen expression by hepatic stellate cells via pathways involving protein kinase A, Src, and extracellular signal-regulated kinases 1/2 signaling cascade or p38 mitogen-activated protein kinase signaling pathway. Mol Pharmacol. 2007 Dec;72(6): 1626-36.
28. Chang W, Wei K, Jacobs SS, Upadhyay D, Weill D, Rosen GD. SPARC suppresses apoptosis of idiopathic pulmonary fibrosis fibroblasts through constitutive activation of beta-catenin. J Biol Chem. 2010 Mar 12;285(11):8196-206.
29. Wang JC, Lai S, Guo X, Zhang X, de Crombrugghe B, Sonnylal S, Arnett FC, Zhou X. Attenuation of fibrosis in vitro and in vivo with SPARC siRNA. Arthritis Res Ther. 2010;12(2):R60.
30. Atorrasagasti C, Aquino JB, Hofman L, Alaniz L, Malvicini M, Garcia M, Benedetti L, Friedman SL, Podhajcer O, Mazzolini G. SPARC down regulation attenuates the profibrogenic response of hepatic stellate cells induced by TGF-betal and PDGF. American journal of physiology. 2011 May;300(5):G739-48.
31. Brekken RA, Sage EH. SPARC, a matricellular protein: at the crossroads of cell-matrix. Matrix Biol. 2000 Dec; 19(7):569-80.
32. Francki A, Bradshaw AD, Bassuk JA, Howe CC, Couser WG, Sage EH. SPARC regulates the expression of collagen type I and transforming growth factor-beta1 in mesangial cells. J Biol Chem. 1999 Nov 5;274(45):32145-52.
33. Camino AM, Atorrasagasti C, Maccio D, Prada F, Salvatierra E, Rizzo M, Alaniz L, Aquino JB, Podhajcer OL, Silva M, Mazzolini G. Adenovirus-mediated inhibition of SPARC attenuates liver fibrosis in rats. J Gene Med. 2008 Sep;10(9):993-1004.
34. Lee MH, Atkinson S, Murphy G. Identification of the extracellular matrix (ECM) binding motifs of tissue inhibitor of metalloproteinases (TIMP)-3 and effective transfer to TIMP-1. J Biol Chem. 2007 Mar 2;282(9):6887-98.
35. Hemmann S, Graf J, Roderfeld M, Roeb E. Expression of MMPs and TIMPs in liver fibrosis - a systematic review with special emphasis on anti-fibrotic strategies. J Hepatol. 2007 May;46(5):955-75.
36. Selman M, Ruiz V, Cabrera S, Segura L, Ramirez R, Barrios R, Pardo A. TIMP-1, -2, -3, and -4 in idiopathic pulmonary fibrosis. A prevailing nondegradative lung microenvironment? Am J Physiol Lung Cell Mol Physiol. 2000 Sep;279(3):L562-74.
37. Divita G, Heitz F, Morris CM, Aldrian-Herrada G,Cell Penetrating Peptides for Intracellular Delivery of Molecules. US Patent 7,579,318. 2009.
38. Rydstrom A, Deshayes S, Konate K, Crombez L, Padari K, Boukhaddaoui H, Aldrian G, Pooga M, Divita G. Direct translocation as major cellular uptake for CADY self-assembling peptide-based nanoparticles. PLoS One. 2011;6(10):e25924.
39. Blackburn MR, Datta SK, Kellems RE. Adenosine deaminase-deficient mice generated using a two-stage genetic engineering strategy exhibit a combined immunodeficiency. J Biol Chem. 1998 Feb 27;273(9):5093-100.
40. Chan ES, Fernandez P, Merchant AA, Montesinos MC, Trzaska S, Desai A, Tung CF, Khoa DN, Pillinger MH, Reiss AB, Tomic-Canic M, Chen JF, Schwarzschild MA, Cronstein BN. Adenosine A2A receptors in diffuse dermal fibrosis: pathogenic role in human dermal fibroblasts and in a murine model of scleroderma. Arthritis Rheum. 2006 Aug;54(8):2632-42.

### ASPECTS OF THE INVENTION:

1. A method of treating a fibrotic or inflammatory condition in an individual, comprising administering to the individual an effective amount of a pharmaceutical composition comprising one or more nucleic acids that modulate the expression of two or more genes involved in the development or progression of fibrosis or inflammation.
2. The method of aspect 1, wherein the two or more genes are selected from the group consisting of CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-κB, and PARP-1 GAD65, sGAD65, BAX, p53 PTEN, STAT5, smoothened, GLI1, GLI2, and Patched-1.
3. A method of treating a fibrotic or inflammatory condition in an individual, comprising administering to the individual an effective amount of a pharmaceutical composition comprising one or more active agents that modulate the activity or expression of two or more genes involved in the development and progression of fibrosis or inflammation, selected from, CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-κB, and PARP-1 GAD65, sGAD65, BAX, p53 PTEN, STAT5, smoothened, GLI1, GLI2, Patched-1.
4. The method of aspect 3, wherein the one or more active agents are nucleic acids.
5. The method of any one of aspects 1, 2, or 4, wherein at least one of the nucleic acids is a single stranded oligonucleotide.
6. The method of any one of aspects 1, 2, or 4, wherein at least one of the nucleic acids is a double stranded oligonucleotide.
7. The method of any one of aspects 1, 2, or 4-6, wherein at least one of the nucleic acids is selected from the group consisting of antisense oligonucleotide, RNAi, shRNA, siRNA, miRNA, or plasmid DNA.
8. The method of aspect 7, wherein at least one of the nucleic acids is RNAi.
9. The method of aspect 8, wherein at least one of the nucleic acids is siRNA.
10. The method of aspect 8, wherein the at least one of the nucleic acids is shRNA.
11. The method of any one of aspects 1, 2, or 4-10, wherein at least one of the nucleic acids is about 10 to about 50 nucleotides long.
12. The method of any one of aspects 1-2 or 4-11, wherein the composition comprises two or more nucleic acids.
13. The pharmaceutical composition of aspect 12, wherein the two or more nucleic acids are of the same kind.
14. The pharmaceutical composition of aspect 12, wherein the two or more nucleic acids are of different kinds.
15. The method of any one of aspects 12-14, wherein the composition comprises two nucleic acids, and wherein the molar ratio of the two nucleic acids is about 0.1:1 to about 10:1.
16. The method of any one of aspects 12-14, wherein the two or more nucleic acids in the pharmaceutical composition are in equal molar proportions.
17. The method of any one of aspects 1, 2, or 4-16, wherein the nucleic acids are associated with a carrier molecule.
18. The method of aspect 17, wherein the nucleic acids are covalently associated with the carrier molecule.
19. The method of aspect 17, wherein the nucleic acids are non-covalently associated with the carrier molecule.
20. The method of aspects 17-19, wherein the carrier molecule is selected from the group consisting of a peptide, a protein, an antibody, a lipid, a phospholipid, a polymer, an aptamer, a nanoparticle, a liposome, a dendrimer, a polymerosome, a viral vector, and a micelle.
21. The method of aspect 20, wherein the carrier molecule is a peptide.
22. The method of aspect 21, wherein the peptide is a cell-penetrating peptide.
23. The method of aspect 22, wherein the cell-penetrating peptide is selected from the group consisting of a PTD-based peptide, an amphipathic peptide, a poly-arginine-based peptide, a MPG peptide, a CADY peptide, Pep-1 peptide or a Pep-2 peptide,.
24. The method of aspects 21 or 22, wherein the nucleic acids and the cell-penetrating peptide are present in a complex.
25. The method of aspect 24 wherein the composition comprises nanoparticles comprising the complexes of the nucleic acids and the cell-penetrating peptide.
26. The method of aspect 25, wherein the average size of the nanoparticles is between 50 and 400 nm.
27. The method of any one of aspects 22-26, wherein the molar ratio of the cell-penetrating peptide and the nucleic acids in the composition is about 100:1 to about 1:50.
28. The method of any one of aspects 1-27, wherein the active agents modulate the activity or expression of two or more genes selected from: SPARC, CTGF, TGFbeta₁, TGFbeta receptor 1, TGFbeta receptor 2, TGFbeta receptor 3, beta-catenin, Adenosine receptor A2A, Adenosine receptor A2B, TIMPs.
29. The method of aspect 28, wherein the active agents modulate the activity of SPARC and TGFbeta1.
30. The method of any one of aspects 1-19, wherein the fibrotic or inflammatory condition is selected from the group consisting of pulmonary fibrosis, idiopathic pulmonary fibrosis, progressive massive fibrosis of the lung, cystic fibrosis, mediastinal fibrosis, liver fibrosis, cirrhosis, endomyocardial fibrosis, cardiac fibrosis, old myocardial infarction, progressive kidney disease, renal fibrosis, myelofibrosis, retroperitoneal fibrosis, nephrogenic systemic fibrosis, Crohn's disease, keloids, scleroderma/systemic sclerosis, arthrofibrosis, adhesive capsulitis, fibromyalgia, peritoneal fibrosis, radiation induced fibrosis, burn induced fibrosis, trauma induced fibrosis, scarring fibrosis, and wound healing fibrosis.
31. The method according to any one of aspects 1-29, wherein the fibrotic or inflammatory conditions is a desmoplastic cancer, wherein the desmoplastic cancer is selected from the group consisting of: squamous cell carcinoma independent of their location, bilio-pancreatic carcinoma, mesothelioma, desmoplastic fibroma, desmoplastic round cell tumor, breast cancer, ovarian cancer, colorectal carcinoma and tumor of gastrointestinal tract, lung cancer, lymphoma, myelofibrosis, leukemia, melanoma, brain tumor (including glioblastoma, cerebral astrocytoma, neuroblastoma, and medulloblastoma), bladder cancer, hepatocellular and urothelial tumor, and tumor of the pituitary gland.
32. The method according to any one of aspects 1-31, wherein said pharmaceutical composition is administered by oral, intravenous, intraarterial, intracardiac, intracatheter, intraperitoneal, intravesical, transdermal, nasal inhalation, pulmonary delivery, intracavity, intracranial, intrathecal, subcutaneous, intradermal, intramuscular, intraocular, topical, rectal, vaginal, direct injection/administration, or local delivery with electrotransfer or microneedle injection.
33. The method according to any one of aspects 1-32, wherein the individual is selected based on the expression level of at least one genes involved in the development and progression of fibrosis or inflammation.
34. The method according to any one of aspects 1-33, further comprising determining the expression level of at least one gene prior to the administration of the pharmaceutical composition.
35. A pharmaceutical composition comprising two or more active agents that modulate the expression of two or more genes involved in the development or progression of fibrosis or inflammation.
36. The pharmaceutical composition of aspect 35, wherein the two or more genes are selected from the group consisting of CTGF(CCN2), TGFbeta₁, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-κB, and PARP-1 GAD65, sGAD65, BAX, p53 PTEN, STAT5, smoothened, GLI1, GLI2, and Patched-1.
37. The pharmaceutical composition of any one of aspects 35-36, wherein the two or more active agents are of the same kind.
38. The pharmaceutical composition of aspect any one of aspects 35-36, wherein the two or more active agents are of different kinds.
39. The pharmaceutical composition of any one of aspects 35-38, wherein the composition comprises two active agents, and wherein the molar ratio of the two active agents is about 0.1:1 to about 10:1.
40. The pharmaceutical composition of any one of aspects 35-39, wherein the two or more active agents in the pharmaceutical composition are in equal molar proportions.
41. The pharmaceutical composition of any one of aspects 35-40, wherein the two or more active agents are nucleic acids.
42. The pharmaceutical composition of aspect 41, wherein at least one of the nucleic acids is a single stranded oligonucleotide.
43. The pharmaceutical composition of aspect 41, wherein at least one of the nucleic acids is a double stranded oligonucleotide.
44. The pharmaceutical composition of any one of aspects 41-43, wherein at least one of the nucleic acids is selected from the group consisting of antisense oligonucleotide, RNAi, shRNA, siRNA, miRNA, or plasmid DNA.
45. The pharmaceutical composition of aspect 44, wherein at least one of the nucleic acids is RNAi.
46. The pharmaceutical composition of aspect 45, wherein at least one of the nucleic acids is siRNA.
47. The pharmaceutical composition of aspect 45, wherein the at least one of the nucleic acids is shRNA.
48. The pharmaceutical composition of any one of aspects 41-47, wherein at least one of the nucleic acids is about 10 to about 50 nucleotides long.
49. The pharmaceutical composition of any one of aspects 41-48, wherein the nucleic acids are associated with a carrier molecule.
50. The pharmaceutical composition of aspect 49, wherein the nucleic acids are covalently associated with the carrier molecule.
51. The pharmaceutical composition of aspect 49, wherein the nucleic acids are non-covalently associated with the carrier molecule.
52. The pharmaceutical composition of any one of aspects 49-51, wherein the carrier molecule is selected from the group consisting of a peptide, a protein, an antibody, a lipid, a phospholipid, a polymer, an aptamer, a nanoparticle, a liposome, a dendrimer, a polymerosome, a viral vector, and a micelle.
53. The pharmaceutical composition of aspect 52, wherein the carrier molecule is a peptide.
54. The pharmaceutical composition of aspect 53, wherein the peptide is a cell-penetrating peptide.
55. The pharmaceutical composition of aspect 54, wherein the cell-penetrating peptide is selected from the group consisting of a PTD-based peptide, an amphipathic peptide, a poly-arginine-based peptide, a MPG peptide, a CADY peptide, Pep-1 peptide, or a Pep-2 peptide.
56. The pharmaceutical composition of any one of aspects 54-55, wherein the nucleic acids and the cell-penetrating peptide are present in a complex.
57. The pharmaceutical composition of aspect 56, wherein the composition comprises nanoparticles comprising the complexes of the nucleic acids and the cell-penetrating peptide.
58. The method of aspect 57, wherein the average size of the nanoparticles is between 50 and 400 nm.
59. The method of any one of aspects 54-58, wherein the molar ratio of the cell-penetrating peptide and the nucleic acids in the composition is about 100:1 to about 1:50.

## Claims

1. A pharmaceutical composition comprising nanoparticles comprising one or more nucleic acids and a cell-penetrating peptide for use in a method of treating a fibrotic or inflammatory condition in an individual, comprising administering to the individual an effective amount of the pharmaceutical composition wherein the one or more nucleic acids inhibit the expression of two or more genes selected from the group consisting of CTGF(CCN2), TGFbeta, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-kB, PARP-1, GAD65, sGAD65, BAX, p53 PTEN, STAT5, smoothened, GLI1, GLI2, and Patched-1.

2. The pharmaceutical composition for use according to claim 1, wherein at least one of the nucleic acids is a) plasmid DNA or b) RNAi, such as siRNA or shRNA.

3. The pharmaceutical composition for use according to claim 1 or 2, wherein at least one of the nucleic acids is about 10 to about 50 nucleotides long.

4. The pharmaceutical composition for use according to any one of claims 1-3, wherein the composition comprises two or more nucleic acids.

5. The pharmaceutical composition according to claim 4, wherein the molar ratio of the two nucleic acids is about 0.1:1 to about 10:1, such as about 1:1.

6. The pharmaceutical composition for use according to any one of claims 1 to 3, wherein the composition comprises three or more nucleic acids.

7. The pharmaceutical composition for use according to any one of claims 1-6, wherein the average size of the nanoparticles is between 50 and 400 nm.

8. The pharmaceutical composition for use according to any one of claims 1-7, further comprising determining the expression level of at least one gene in the individual prior to the administration of the pharmaceutical composition.

9. A pharmaceutical composition comprising nanoparticles comprising two or more nucleic acids and a cell-penetrating peptide, wherein the two or more nucleic acids inhibit the expression of two or more genes selected from the group consisting of CTGF(CCN2), TGFbeta, TGFbeta receptor 1, TGFbeta receptor 2, TGFbeta receptor 3, beta-catenin, SPARC, VEGF, Angiotensin II, TIMP, HSP47, thrombospondin, CCN1, LOXL2, MMP2, MMP9, CCL2, Adenosine receptor A2A, Adenosine receptor A2B, Adenylyl cyclase, Smad 3, Smad 4, Smad 7, SOX9, arrestin, PDCD4, PAI-1, NF-kB, PARP-1, GAD65, sGAD65, BAX, p53 PTEN, STAT5, smoothened, GLI1, GLI2, and Patched-1.

10. The pharmaceutical composition of claim 9, wherein the composition comprises two nucleic acids, and wherein the molar ratio of the two nucleic acids is about 0.1:1 to about 10:1, such as about 1:1.

11. The pharmaceutical composition of claim 9 or 10, wherein at least one of the nucleic acids is a) plasmid DNA or b) RNAi, such as siRNA or shRNA.

12. The pharmaceutical composition of any one of claims 9-11, wherein at least one of the nucleic acids is about 10 to about 50 nucleotides long.

13. The pharmaceutical composition of any one of claims 9-12, wherein the average size of the nanoparticles is between 50 and 400 nm.

14. The pharmaceutical composition of claim 13, wherein the molar ratio of the cell-penetrating peptide and the nucleic acids in the composition is about 100:1 to about 1:50.
